(19) Europäisches Patentamt
European Patent Office
Office européen des brevets

(11) **EP 1 450 960 B1**

(12) **EUROPEAN PATENT SPECIFICATION**

(45) Date of publication and mention
of the grant of the patent:
**17.08.2011  Bulletin 2011/33**

(51) Int Cl.:
*B03C 1/00* *(2006.01)*     *B03C 1/025* *(2006.01)*
*B03C 1/30* *(2006.01)*     *G01N 33/543* *(2006.01)*
*C12Q 1/68* *(2006.01)*

(21) Application number: **02790431.7**

(22) Date of filing: **25.11.2002**

(86) International application number:
**PCT/EP2002/013213**

(87) International publication number:
**WO 2003/045565 (05.06.2003 Gazette 2003/23)**

(54) **APPARATUS AND METHOD FOR MODIFICATION OF MAGNETICALLY IMMOBILIZED BIOMOLECULES**

APPARAT UND VERFAHREN ZUR MODIFIZIERUNG VON MAGNETISCH INMMOBILIZIERTE BIOMOLEKULEN

APPAREIL ET PROCEDE PERMETTANT DE MODIFIER DES BIOMOLECULES IMMOBILISEES MAGNETIQUEMENT

(84) Designated Contracting States:
**AT BE BG CH CY CZ DE DK EE ES FI FR GB GR IE IT LI LU MC NL PT SE SK TR**

(30) Priority: **26.11.2001   US 994487**

(43) Date of publication of application:
**01.09.2004   Bulletin 2004/36**

(73) Proprietor: **Miltenyi Biotec GmbH**
**51429 Bergisch Gladbach (DE)**

(72) Inventors:
• **MILTENYI, Stefan**
**51429 Bergisch Gladbach (DE)**
• **KÖHLER, Matthias**
**69514 Laudenbach (DE)**

(74) Representative: **Brasnett, Adrian Hugh et al**
**Mewburn Ellis LLP**
**33 Gutter Lane**
**London**
**EC2V 8AS (GB)**

(56) References cited:
**EP-A- 1 081 234      WO-A-01/17687**
**US-A- 3 869 390**

**Description**

**FIELD OF THE INVENTION**

The present invention relates to the use of high gradient magnetic separation (HGMS) techniques and apparatus for modifying biomolecules *in situ.*

**BACKGROUND OF THE INVENTION**

High gradient magnetic separation refers to a procedure for selectively retaining magnetic materials in a chamber or column disposed in a magnetic field. This technique can also be applied to non-magnetic targets labeled with magnetic particles. In one application of this technique a target material, typically a biological material, is labeled by attaching the target material to a magnetic particle. The attachment is generally through association of the target material with a specific binding partner which is conjugated to a coating on the particle which provides a functional group for the conjugation. Other kinds of attachments have been described. For example, colloidal magnetic dextran iron particles can be selectively bound to a target cell through use of a bi-specific antibody having specificity for both dextran and for a target cell surface antigen. Lansdorp and Thomas (1990) Mol. Immunol. 27:659. In addition, direct chemical or physical association of a magnetic particle with a target material is possible.

The material of interest, thus coupled to a magnetic "label", is suspended in a fluid which is then applied to the chamber. In the presence of a magnetic gradient supplied across the chamber, the magnetically labeled target is retained in the chamber; if the chamber contains a matrix, it becomes associated with the matrix. Materials which do not have magnetic labels pass through the chamber. The retained materials can then be eluted by changing the strength of, or by eliminating, the magnetic field. Alternatively, retained particles can be eluted by supplying magnetized fluid. U.S. Patent No. 5,411,863. The magnetic field can be supplied either by a permanent magnet or by an electromagnet. The selectivity for a desired target material is supplied by the specific binding-partner conjugated to the magnetic particle, by direct chemical conjugation or physical association of the magnetic particle with the target. The chamber across which the magnetic field is applied is often provided with a matrix of a material of suitable magnetic susceptibility to induce a high magnetic field gradient locally in the chamber in volumes close to the surface of the matrix. This permits the retention of fairly weakly magnetized particles, and the approach is referred to as high gradient magnetic separation (HGMS).

Various methods have been described for capturing cells or molecules using magnetic separation. U.S. Pat. No. 4,452,773 describes the preparation of magnetic iron-dextran microspheres and provides a summary of art describing the various means of preparation of particles suitable for attachment to biological materials. U.S. Pat. No. 4,230,685 describes an improvement in attaching specific binding agents to the magnetic particles wherein a particle coated with an acrylate polymer or a polysaccharide can be linked through, for example, glutaraldehyde to a preparation of protein A which can then selectively bind antibodies through the Fo portion, leaving the immunoreactive Fab regions exposed. U.S. Patent No. 6,020,210 describes the use of HGMS to retain cells. U.S. Patent No. 6,159,378 describes a method for handling magnetic particles in a fluid. WO 01/17687 describes the use of HGMS techniques in the process of modifying selected cells. U.S. Patent No. 6,159,689 describes a method of capturing a molecule using magnetic particles.

Various forms of apparatus for use in HGMS have also been described. U.S. Pat. No. 4,738,773 describes a separation apparatus which employs helical hollow tubing made either of stainless steel or TEFLON™ (polytetrafluoroethylene) for example, wherein the helices are placed in an applied magnetic field. U.S. Pat. No. 4,664,796 describes configurations in which the position of the magnetic field can be varied across the separation column, Kronick, U.S. Pat. No. 4,375,407 describes a device for HGMS in which the fluid, which contains the particles to be separated, is passed through a filamentary material that has been coated with a hydrogel polymer.

Various methods are currently available for modifying biological molecules *in vitro.* In many of these methods, the temperature at which modification occurs must be carefully controlled. Controlled temperature conditions are important in many instances because the structure of the molecule being modified, a reactant in a modification reaction, or an interaction between two molecules, is temperature-dependent or temperature sensitive. Frequently, reactants must be separated from products or intermediates, and this is generally accomplished by washing (e.g., phenol/chloroform precipitation of DNA), or by column separation (e.g., gel filtration) after the modification reaction. Furthermore, when two or more modification reactions must be carried out on a single molecule, or when modification of one molecule results in generation of a second molecule, then modification of the second molecule, a separation step is frequently carried out between the two modification steps.

One drawback to such methods is that transfer of material from reaction vessel to separation column (or other separation apparatus) is required, which inevitably results in loss of product. This drawback is a particular problem when product is in limited amounts. Furthermore, such methods are time consuming, laborious, and do not generally lend themselves to automation.

There is a need in the art for improved apparatus and methods for modifying biological molecules under temperature-controlled conditions, without the need for multiple transfer steps. The present invention addresses this need.

Literature

U.S. Patent Nos. 5,711,871; 5,705,059; 5,691,208; 5,543,289; 5,779,892; and 6,020,201.

**SUMMARY OF THE INVENTION**

The present invention provides methods of modifying a biological molecule ("a biomolecule") using a high gradient magnetic separation (HGMS) system. The method generally involves immobilizing a magnetically labeled biomolecule on a magnetic separation device by applying a magnetic field to the labeled biomolecule when it is in the separation device, and modifying the magnetically immobilized biomolecule. In many embodiments, one or more modification steps are conducted under temperature-controlled conditions. In some embodiments, the method further comprises the step of separating a reaction product from an immobilized biomolecule. In other embodiments, the method further comprises the step of eluting the modified biomolecule from the column. In still other embodiments, the method comprises conducting two or more modification steps, optionally with intervening elution and/or washing steps.

In still other embodiments, the method comprises conducting at least one modification step on a magnetically immobilized biomolecule, and capturing the modified or newly synthesized biomolecule on a second binding moiety in the magnetic separation device, optionally with intervening elution and/or washing steps. After capturing the modified or newly synthesized biomolecule, the modified or newly synthesized biomolecule is further modified and/or purified.

An advantage of the instant methods is that modification and separation steps, as well as purification and washing steps, can be carried out in a single device (or within a single unit within the device, e.g., a chamber within the device), thereby avoiding the drawbacks associated conventional purification of non-immobilized modified target material, such as loss of product.

A further advantage of the instant methods is that an enzyme used in modification can be washed away from the column once the modification is completed. Accordingly, no inactivation steps or additional purification steps are necessary, thereby saving time and reducing loss of product. In addition, no toxic reagents used in standard protocols for removing enzymes are needed. Thus, the final product has increased purity.

The invention further provides for modifying a magnetically immobilized biomolecule, whereby the biomolecule is immobilized in suspension. The strength of the magnetic field that is applied to the separation device can be adjusted to provide for the formation of a suspension of the magnetic particles with which the biomolecules are associated. Depending on the strength of the applied magnetic field, biomolecules can be fixed in place, or can be in a suspension. Keeping the biomolecules in suspension is advantageous for some applications, where homogeneous modification of the biomolecules is desired. The suspension can be localized, e.g., in certain high magnetic field or gradient areas of the matrix; or throughout the entire void volume of the separation device.

A further advantage is that the temperature under which a modification reaction can be controlled, and altered according to the specific reaction being carried out.

The present invention includes various arrangements for controlling the temperature of columns placed in an HGMS separation system. A separation unit is provided with a controllable heat source for controlling the temperature within at least one separation chamber in situ. Each separation chamber may contain a wettable, flow through heat conducting matrix. Alternatively, or additionally, the separation unit may be provided with a controllable cooling source coupled to each location where a separation chamber is to be mounted. In several examples, heating and cooling functions are performed by the same controllable source(s).

A controller may be provided which couples each controllable heat/cooling source with a power source, such that the controller functions to control an amount of power delivered to each controllable source to control a temperature thereof.

One or more feedback sensors may be associated with the controllable heat/cooling sources to provide feedback to the controller regarding temperatures of the controllable heat/cooling sources.

As an alternative to providing heating and/or cooling mechanisms within a separation unit, the present disclosure further provides various external temperature regulating units adapted to interface with an HGMS separation unit and control the temperature of columns held thereby. An example of an external regulating unit includes a base portion, finger elements extending from the base portion and adapted to fit within slots in the HGMS separation unit which hold the separation columns, and a controllable heating element at an end of each finger element, adapted to apply a controlled amount of heat/cooling to the separation column in the gap, respectively.

The base portion may comprise a heat conductor made of a heat conducting material. The finger elements may be formed of the same heat conducting material as the heat conductor,

These and other objects, advantages, and features of the invention will become apparent to those persons skilled in the art upon reading the details of the apparatus and methods, as more fully described below.

## BRIEF DESCRIPTION OF THE DRAWINGS

Fig.1 is a plan view of an example of a HGMS system according to the present invention.

Fig. 2 is a sectional view of a micro column taken along line 2-2 in Fig. 1.

Fig.3A is a schematic, partial sectional view of an example of a HGMS system employing a heating film according to the present invention.

Fig.3B is an enlarged view of that portion of Fig. 3A outlined by reference numeral 3.

Fig. 4A is a schematic, partial sectional view of another example of a HGMS system employing a heating film according to the present invention.

Fig. 4B is an isolated view of a metal sheet used in the embodiment of Fig. 4A.

Fig. 5A is a schematic, partial sectional view of another example of a HGMS system according to the present invention, this example employing power resistance type heating.

Fig. 5B is an isolated top view of a heat conductor used in the embodiment of Fig. 5A.

Fig. 5C is an isolated side view of a heat conductor used in the embodiment of Fig. 5A.

Fig. 6A is a partial sectional view of an external regulating unit employing Peltier type heating/cooling and engaged with an HGMS unit according to the present invention.

Fig. 6B is an end view of the external regulating unit of Fig. 6A.

Fig. 7A is a schematic, partial sectional view of an example of a HGMS system employing Peltier heating elements according to the present invention.

Fig. 7B is a sectional view of the embodiment shown in Fig. 7A taken along line 7-7.

Fig. 8A is a schematic, partial sectional view of another example of a HGMS system employing Peltier heating elements according to the present invention.

Fig. 8B is a sectional view of the embodiment shown in Fig. 8A taken along line 8-8.

Fig. 9A is a partial sectional view of an HGMS system with pneumatic heating and cooling according to the present invention.

Fig. 9B is a sectional view of the embodiment shown in Fig. 9A, taken along line 9-9.

Fig. 10 is a partial sectional view of an HGMS system with hydraulic heating/cooling according to the present invention.

Fig. 11 is a partial sectional view of an HGMS system with radiant heating according to the present invention.

Fig. 12 is a partial sectional view of an HGMS system employing inductive heating according to the present invention.

## DEFINITIONS

As used herein, the term "biomolecule" refers to any molecule derived from a biological source, including synthetic molecules that are not normally associated with a biological entity, but are modifications or analogs of molecules normally associated with a biological entity (e.g., an animal, a plant, a eubacterium, an archaebacterium, a fungus, a mold, a yeast, an algae, and the like). The term "biomolecule" further encompasses a plurality of biomolecules, which may be heterogeneous or homogeneous. As such, the term further encompass libraries of synthetic and semi-synthetic analogs of biomolecules. Biomolecules include, but are not limited to, polynucleotides; polypeptides; polysaccharides; lipids; molecules that comprise one or more of a polynucleotide, a polysaccharide, a lipid, and a polypeptide, including, but not limited to, lipopolysaccharides, lipoproteins, glycolipids, glycoproteins, proteoglycans, peptide nucleic acids, and the like. A biomolecule may comprise one or more modifications, including, but not limited to, acylations, acetylations, phosphorylations, addition of sulfur groups, and the like.

The terms "polynucleotide" and "nucleic acid molecule" are used interchangeably herein to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes single-, double-stranded and triple helical molecules. "Oligonucleotide" generally refers to polynucleotides of between about 5 and about 100 nucleotides of single- or double-stranded DNA. However, for the purposes of this disclosure, there is no upper limit to the length of an oligonucleotide. Oligonucleotides are also known as oligomers or oligos and may be isolated from genes, or chemically synthesized by methods known in the art.

The following are non-limiting embodiments of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. A nucleic acid molecule may also comprise modified nucleic acid molecules, such as methylated nucleic acid molecules and nucleic acid molecule analogs. Analogs of purines and pyrimidines are known in the art. Nucleic acids may be naturally occurring, *e.g.* DNA or RNA, or may be synthetic analogs, as known in the art. Such analogs may be preferred for use as probes because of superior stability under assay conditions. Modifications in the native structure, including alterations in the backbone, sugars or heterocyclic bases, have been shown to increase intracellular stability and binding affinity. Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O'-5'-S-phosphorothioate, 3'-S-5'-O- phosphorothioate, 3'-CH2-5'-O-phosphonate and 3' NH-5'-O-phosphoroamidate. Peptide nucleic acids replace the entire ribose phosphodiester backbone with a peptide linkage.

Sugar modifications are also used to enhance stability and affinity. The $\alpha$-anomer of deoxyribose may be used, where the base is inverted with respect to the natural $\beta$-anomer. The 2'-OH of the ribose sugar may be altered to form 2'-O methyl or 2'-O-allyl sugars, which provides resistance to degradation without compromising affinity.

Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5- propynyl-2'-deoxyuridine and 5-propynyl-2'-deoxycytidine have been shown to increase affinity and biological activity when substituted for deoxythymidine and deoxycytidine, respectively.

The terms "polypeptide" and "protein," used interchangeably herein, refer to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

As used herein, a "selected biomolecule" (also referred to as a "target material") is a biomolecule the practitioner desires to modify. The biomolecule may bear some characteristic that differentiates it from other biomolecules in a heterogeneous suspension, and that will allow it to be labeled with a magnetic particle, immobilized, and modified. However, the biomolecule need not be separated from other biomolecules in all applications. According to the invention, the selected biomolecule is retained on an HMG column, and modified while retained on the column. The selected biomolecule that has been modified can then be eluted. Alternatively, the modification results in generation of at least a second biomolecule, which second biomolecule is then eluted.

"Retention" of a selected biomolecule ensures that the selected biomolecule remains in the device (or chamber within the device) while unwanted biomolecules are removed. Typically, the retention of the selected biomolecule is by immobilization.

As used herein, "immobilizing" a selected biomolecule in a magnetic cell separation refers to retention of the biomolecule in the column in a substantially fixed position. The term "substantially fixed" refers to the fact that the biomolecule remains in the column at a position, which position may vary substantially over time, depending on the strength of the applied magnetic field. Thus, e.g., the applied magnetic field can allow for movement of the biomolecule within the area of the applied magnetic field.

"Removing" a selected biomolecule from a magnetic cell separation column involves eluting the selected biomolecule subsequent to retention or immobilization, with or without the magnetic label. In situations where a high purity of selected biomolecules is desired, the selected biomolecule may be removed and resuspended in a suitable buffer. Alternatively, a selected biomolecule may be removed and returned to the original suspension after modification of the selected biomolecule in the device as described herein. Removing at least a second biomolecule generated as a result of modification of a selected biomolecule involves eluting the second biomolecule subsequent to its production.

The terms "conjugated," "attached," and "linked" (and similar terms, e.g. "conjugation," "attachment," and "linkage") are used interchangeably herein to refer to a chemical association of two molecules, e.g., a nucleic acid molecule and a polypeptide. The chemical association may be covalent or non-covalent. The two molecules can be linked directly, or indirectly, e.g., via a linker ("spacer") molecule, a solid support, and the like.

As used herein, "labeling" is the process of affixing a marker to a biomolecule, allowing, sometimes after further processing, those biomolecules to be separated from a heterogeneous suspension and/or detected, analyzed or counted. Labels can be specifically targeted to selected biomolecules, but need not be. Such markers or labels include, but are not limited to, colored, radioactive, fluorescent, or magnetic molecules or particles conjugated to antibodies or other biological molecules or particles known to bind to a particular biomolecule or class of biomolecule. Other biologically reactive label components that can serve as alternatives to antibodies include, but are not limited to, genetic probes, lipids, proteins, peptides, amino acids, sugars, polynucleotides, enzymes, coenzymes, cofactors, antibiotics, steroids, hormones or vitamins.

As used herein, "magnetically labeling" a biomolecule refers to affixing a magnetic label to the biomolecule, such labeling being accomplished by affixing a particle or molecule with magnetic properties to said biomolecule. Magnetic labels comprising an antibody, a protein, or a nucleic acid molecule conjugated to a magnetic particle are commercially available from Miltenyi Biotec GmbH (Friedrich Ebert Str. 68, D-51429 Bergisch Gladbach, Germany). Such a label can optionally include a fluorescent or radioactive particle or component as well.

Methods to prepare superparamagnetic particles are described in U.S. Pat. No. 4,770,183. With respect to terminology, as is the general usage in the art:

"Diamagnetic" as used herein, and as a first approximation, refers to materials which do not acquire magnetic properties even in the presence of a magnetic field, i.e., they have no appreciable magnetic susceptibility.

"Paramagnetic" materials have only a weak magnetic susceptibility and when the field is removed quickly lose their weak magnetism. They are characterized by containing unpaired electrons which are not coupled to each other through an organized matrix. Paramagnetic materials can be ions in solution or gases, but can also exist in organized particulate form.

"Ferromagnetic" materials are strongly susceptible to magnetic fields and are capable of retaining magnetic properties when the field is removed. Ferromagnetism occurs only when unpaired electrons in the material are contained in a crystalline lattice thus permitting coupling of the unpaired electrons. Ferromagnetic particles with permanent magnetization have considerable disadvantages for application to biological material separation since suspension of these particles easily aggregate due to their high magnetic attraction for each other.

"Superparamagnetic" materials are highly magnetically susceptible, e.g., they become strongly magnetic when placed in a magnetic field, but rapidly lose their magnetism. Superparamagnetism occurs in ferromagnetic materials when the crystal diameter is decreased to less than a critical value. Superparamagnetic particles are preferred in HGMS.

Although the above-mentioned definitions are used for convenience, it will immediately be apparent that there is a continuum of properties between paramagnetic, superparamagnetic, and ferromagnetic, depending on crystal size and particle composition. Thus, these terms are used only for convenience, and "superparamagnetic" is intended to include a range of magnetic properties between the two designated extremes.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a biomolecule" includes a plurality of such biomolecules and reference to "the modification" includes reference to one or more modifications and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

## DETAILED DESCRIPTION OF THE INVENTION

The invention provides an apparatus and method for modification of magnetically immobilized biomolecules, particularly for temperature-controlled modifications.

## HIGH GRADIENT MAGNETIC SEPARATION SYSTEM

The present invention provides HGMS systems for modification of magnetically immobilized biomolecules. Each system includes at least one separation/modification chamber which may be designed as a separation column with an open inlet and an outlet, but could also be formed as a single cup or multiwell cup or the like. A temperature regulating device is provided, and a heat conducting matrix (which may optionally be internally magnetizable) is provided in each separation chamber to provide heating or cooling to the contents within the chamber for controlling the temperature thereof during processing. In this way, the temperature of a liquid phase in a separation chamber can be monitored, controlled and changed at will during processing. The temperature control range of the devices of the present invention is from about -30° C to about 100° C, from about 4° C to about 65° C, or from about 12° C to about 42° C.

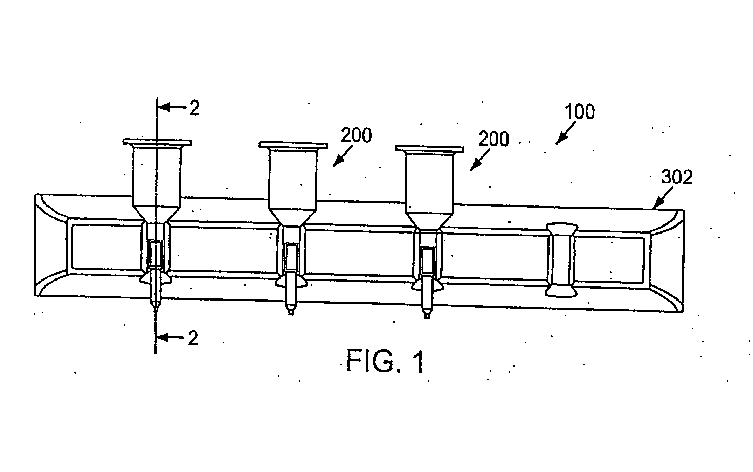

Fig. 1 shows an example 100 of a HGMS system according to the present invention. Although the example shown is of a micro column system having a separation unit 302 adapted to hold a plurality of micro columns 200, it is noted that the present invention is applicable to single column embodiments, as well as to single and multiple column systems (e.g., 4 separation chamber embodiment compatible to the heated MicroMACS™ as shown in Figs. 1 and 3A-12; or injection molded part with 8, 12 or 96 separation chambers compatible with 96 multiwell plat format), and may also employ larger columns Also, as mentioned above, the invention is applicable to systems employing one or more separation chambers that are configured other than in the form of a separation column, e.g., cup-shaped chambers and the like which have an input, but not an output for flow through. Further the invention is applicable to automated systems.

Each micro column 200 shown Fig. 1 is configured to optimize the processing of small volume samples of biomolecules of the type described below. The micro column 200 is substantially reduced in void volume while maintaining optimal flow speeds and is designed for separation of biomolecules that are magnetically bound by specific biological/chemical interactions, from other molecules or cells in a high gradient magnetic field, and for elution of the biomolecules in a small volume.

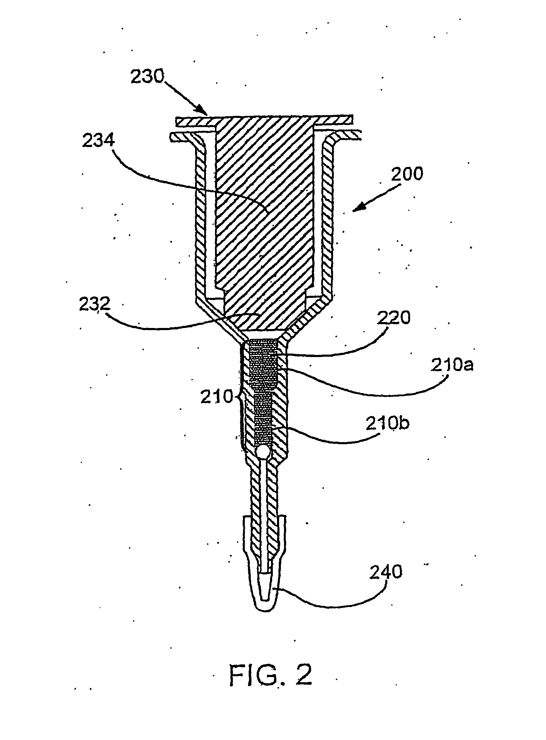

Referring to Fig. 2, a matrix 210 is provided in the separation chamber of the column 200, and in this example, has a larger diameter portion 210a and a smaller diameter portion 210b beneath the larger diameter portion. Columns of this configuration are described at greater length in commonly assigned co-pending application Serial No. 09/556,179, titled "Magnetic Micro Separation Column and Method of Using It", filed April 20, 2000, which is hereby incorporated by reference thereto, in its entirety. The matrix 210 contains ferromagnetic material 220, such as balls, particles, steel wool, or other integrated, three dimensional mesh having the desired porosity. The ferromagnetic material may be coated with a coating, such as lacquer, or the like, to maintain the relative positioning of the balls or particles in the matrix. Meshes or frits may also be used in addition to, or alternative to the coating to maintain relative positioning. The balls or particles have a size greater than about 100 microns, or greater than about 200 microns and less than about 1000 microns.

The micro column may be made hydrophilic by manufacturing it from a hydrophilic plastic, or by coating it interiorly with a hydrophilic material such as polyvinyl pyrrolidone, for example. A coating, if used, may prevent release of substances of the matrix into the solution and/or may optionally be designed to release substances from the coating itself which would be useful for the conduct of a separation or modification process. Preferably, a coating used will be inert to unspecific binding of biomolecules. Optionally, it may be specific to binding of certain biomolecules. For example, an antigen-specific antibody or a ligand can be included on the matrix to remove a modifying agent, such as an enzyme. As one non-limiting example, the matrix can include an antibody specific for a modifying enzyme such that the modifying enzyme is not eluted along with the product of the modification reaction. As another alternative, a detergent buffer may be used in columns exhibiting less hydrophilicity. Buffers which are poured into the micro column may also include surfactants, such as sodium dodecyl sulfate (SDS), for example.

Each column may optionally include an inlet port seal 230 and an outlet port seal 240 for making the separation chamber into a closed system during processing. The provision of such a closed system will eliminate or significantly reduce evaporation of the liquids in the separation chamber during incubation steps at increased temperature, for example. The inlet port seal 230 may be a single use or reusable plug. The inlet port seal 230 may be a sealing part that is inserted only during incubation and them removed during all other process steps such as washing, elution, etc., for example. The inlet port seal may include the sealing part just described, in addition to spheres, conical or cylindrical parts or one or more filters or meshes that remain in place when the sealing part is removed, and that act to reduce evaporation, although they do not completely seal the inlet. As a further alternative, the inlet port seal may stay permanently in position at the inlet of the separation chamber and may comprise one or more filters, meshes, small spheres/particles (e.g., glass spheres), any cover with one or more small holes therethrough, a membrane which is pierceable with a needle when fluid is applied through a needle and into the separation chamber, and the like. A single use inlet port seal 230 may be made of a plug 234 of metal, such as stainless steel, or plastic and is preferably an injection molded thermoplastic which is then provided with a sealing tip 232 made of Para-film, silicone rubber, an elastomer or a thermoplast, for example. In another example, a single use inlet port seal 230 comprises an injection molded thermoplastic plug 234 with an integrated sealing tip 232 formed of a silicone rubber, an elastomer or a thermoplast that is preferably of the same material as the injection molded thermoplastic plug 234 or is injection molded together with the plug 234 in a two component molding process.

A reusable inlet port seal 230 may be formed as a plug 234 of metal such as stainless steel or plastic provided with a sealing tip 232 of Para-film, silicone rubber, an elastomer or a thermoplast, for example, having properties that can withstand cleaning solutions and/or sterilization procedures and still function properly thereafter. Alternatively, a reusable plug 234 as described above may be provided with a single use cover of Para-film, silicone rubber, an elastomer or a thermoplast, for example, which can be replaced prior to each use.

Further, inlet port seal 230 may be a fluidic cover which could be a suitable oil, such as mineral oil, or other liquid having a boiling point higher than that of water and which is biocompatible (e.g., glycerine, oils, alkanes, mixtures thereof and the like) for example, that acts to significantly reduce or eliminate evaporation, but will at the same time allow the addition of fluids therethrough. Depending upon the density of the added fluid, the fluidic cover may "swim up" on top of the added fluid or be driven through the column. The optional outlet port seal 240 may also be for single use or reusable. Both single use and reusable outlet port seals may be formed as a rubber sleeve with one end closed, rubber sleeve with one closed end with the closed end penetrated by the outlet of the column and slidable over the end of the coloumn to close the outlet during incubation procedures, a silicone rubber cap, and elastomer sheet (e.g., Parafilm), any cup-formed plug, or the like. Suitable materials for forming the outlet port seal 240 include rubber, latex, polytetrafluoroethylene, other polymers which are inert to materials being processed, injection molded foam, stainless steel, other relatively inert and biocompatible metals, sheets or foils of the previously mentioned metals, fluidic covers made from glycerine or other biocompatible oils, and the like. The outlet port seal 240 may function not only to seal the outlet, but additionally to provide protection from accidentally breaking the outlet of the column.

A high gradient magnetic field is generated in the matrix 210 upon placing it into a magnetic field. Thus, when the column 200 is positioned in separation unit 302, the magnets on opposite sides of the matrix 210 supply the magnetic field to generate a high gradient magnetic field in matrix 210. The matrix 210 readily demagnetizes when it is taken out of the magnetic field. When in the magnetic field, the magnetized particles of the matrix 210 retains single superparamagnetic MicroBeads and material (i.e., "biomolecules") attached to them from a solution or reaction mixture of variable viscosity which is inputted into the column 200, thus immobilizing the biomolecules of interest. Once immobilized, further processing of the biomolecules can be conducted *in situ* in the separation chamber. Upon the completion of processing (or at least one phase of processing), the immobilized biomolecules can be released when the application of the magnetic field to the matrix 210 is reduced in strength or eliminated altogether, thereby releasing the superparamagnetic MicroBeads from the matrix 210.

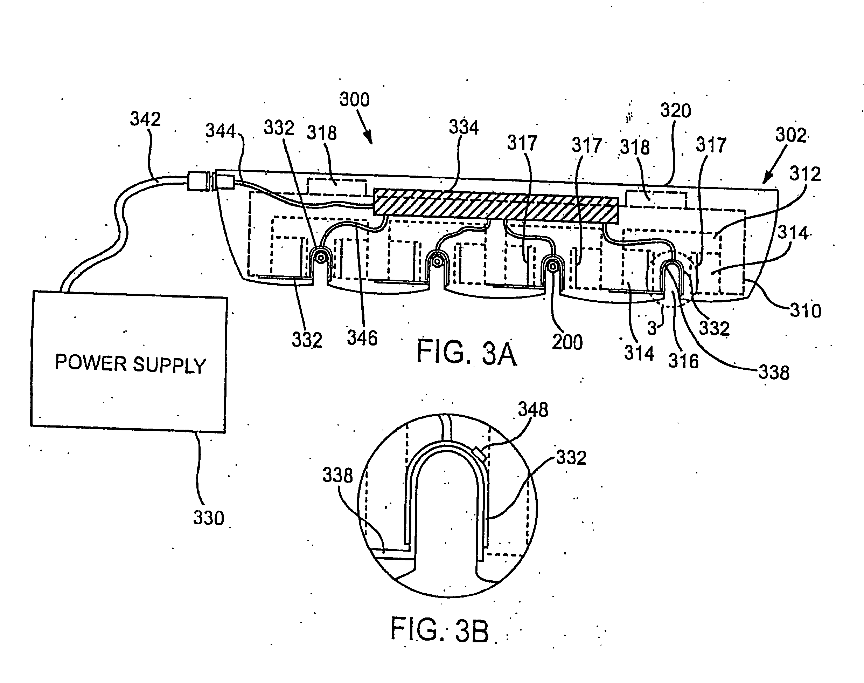

Referring to Fig. 3A, a partial sectional view of an HGMS system 300 with heating capability is shown. The separation unit 302 includes a yoke 310 that forms the basic framework of the unit and that concentrates the magnetic fields. The yoke is configured to include a notch 312 in each area where a column is to be received. A pair of magnets 314 are mounted in each notch 312 so as to form a narrower gap 316 where the magnetic field of the magnets is focused and where a micro column 200 is to be received for carrying out HGMS procedures.

Two magnets 318 may be mounted to the back of the yoke 310 to facilitate attachment or mounting of the separation unit 302 to a ferromagnetic device such as an iron stand. Of course a different number of magnets 318 may be used. Additionally or alternatively, other mounting means such as clamps, screws, bolts, adhesive, etc, could be used to mount the unit.

In this embodiment, a heating element comprises a heating film 332 which is provided in each gap 316 so as to at least partially surround, and optionally, completely surround, the column 200 when it is positioned for processing, to provide heat thereto. Each heating film 332 is thermally connected (by gluing, for example) to a heat conductor 338 as seen best in Fig. 3B. Each heat conductor 338 may be a thin sheet of metal, such as aluminum or other metal with good heat conducting properties. The heat conductors both conduct the heat provided by the heating film 332 and work to evenly apply the heat around the column 200.

In embodiments where only heating will be required, heating elements 332 may be heating films with a specially designed electrical resistance to achieve the desired temperature ranges. Examples of the heating film include Kapton insulation, silicone rubber insulation, transparent heating film with Mylar insulation (which is used for LCD heating), and the like. A flexible film is formed from one of the foregoing materials, for example, and a meandering metallic film circuit is coated on the film to form a resistive circuit. An effective heating area of a heating element 332 is typically in the range of about 0.3 to 36 $cm^2$ and an electrical resistance is typically in the range of about 5.5 to 360 ohms. This type of heating arrangement is advantageous in that it takes up very little space, is easily tailored to customer specifications with regard to resistance and heat range capability, and requires relatively low power. A specific example of one such heating element is one produced by Telemeter Electronic GmbH, 86609 Donauwörth, Germany, Model HK-913-H, Art.-No. 33/584, Spec: 150,15x48 mm, 1.4 $cm^2$ effective heating area.

The heating elements 332 are thermally coupled with heat conducting elements 338, which in turn, are thermally coupled with magnets 314, as well as with the columns 200 when the columns are mounted in the separation unit 302. The heat conducting elements may be formed of materials possessing a high coefficient of heat transfer, such as copper, (anodized) aluminum, stainless steel or other metal having sufficient heat conducting properties, and are shaped so as to closely contact the columns where they interface with the columns.

Optionally, an insulation layer 317 may be provided between magnets 314 and heating element 332 to prevent excessive heating of the magnets 314, as generation of magnetic fields tends to deteriorate if the magnets reach a temperature greater than about 100°C. The insulation layer may be formed of a porous polymer or other material which would effectively prevent overheating of the magnets 314. At least the surfaces of the magnets that directly interface with the heating elements 332 may be covered, and further optionally, all surfaces of the magnets 314 may be covered with an insulation layer 317. Although this feature is only shown in Figures 3A and 3B, it is not to be limited only to the embodiment shown in those figures; it may be equally applied to other embodiments described herein and to those covered by the claims.

An electronic control board 334 is provided in the unit 302 or as an external unit and is electrically connected to each of the heating/cooling elements or sources 332 and an external power supply 330, which is capable of supplying sufficient electrical power to generate the amount of heat needed and to control the temperature as well as the rate of heat generation, and which is adjustable to provide suitable power for the control board 334. The power level may be further increased or decrease by the electronic control board 334. An exemplary power supply is available from Schuricht GmbH & Co. KG, Bremen, Germany, Model SSL40-7612 12C/3A, characterized by 40W, 100-250V AC input, 12V output. A power cord 342 connecting the power supply 330 to the unit 302 may be releasably coupled to a power cord 344 leading to the electronic control board 334, by any of a number of well known jack couplers having a male and a female component. The heating films 332 are independently connected to the electronic board 334 by respective electrical conductors 346 to allow independent temperature control over each source 332 by the control board 334.

A feedback sensor 348, such as a thermocouple, for example, may be provided on at least one heating element, to as many as one on each heating element 332, at the interface with the heat conducting elements 338 or on the surfaces of the heat conducting elements that contact the columns, for example, and are electrically connected to the control board 334 to feed back signals which are representative of the temperature measured by each thermocouple. The control board 334 includes a temperature control unit/regulator or attemperator (e.g., microprocessor, bimetal relay) which receives inputs from the thermocouples and converts them to temperature readings, compares the temperatures to temperature settings which are either manually inputted to the control board by an operator, or programmed in, and determines whether heating, cooling or stasis of each individual source is required. The appropriate action is taken by controlling the amount of power inputted from the power supply 330. Programmed routines may be stored in the control board for automatically controlling the temperature of each heating/cooling source for various processes and cycles of treatment as described below.

The unit is entirely encased in a non-fragile covering or housing 320 which holds the columns in correct positions and protects the internal components of the unit 302, as well as making the unit more visually appealing and easier to clean/sterilize. The covering 320 may be a closed cell foam, plastic hard foam cover of a resin such as polyurethane resin, injection molded thermoplastic, aluminum/stainless steel milled, or the like, for example.

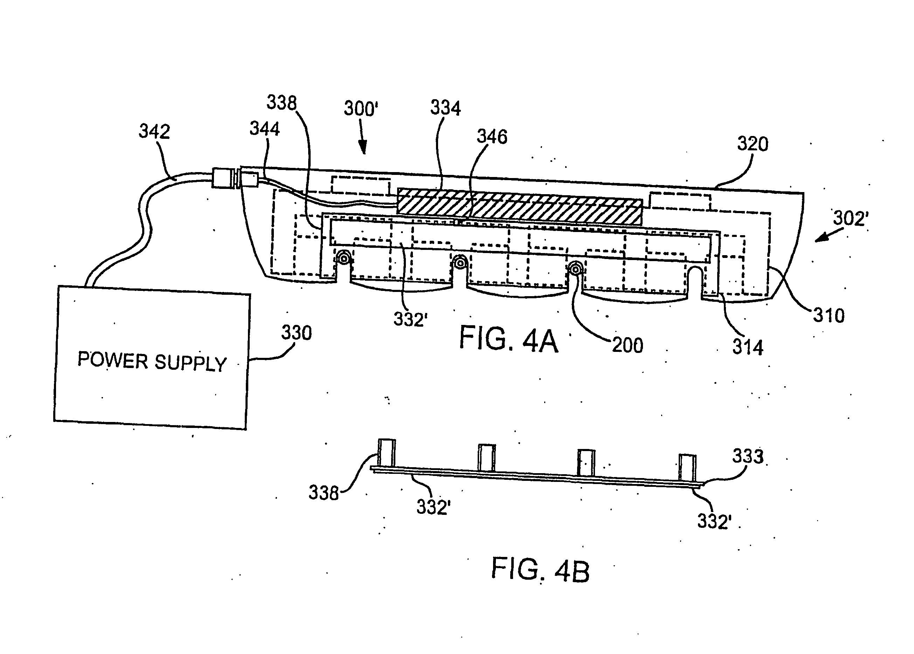

Fig. 4A shows a variation of a system 300' which employs a separation unit 302' having a heating film 332' arranged slightly differently from that in Fig. 3A. In this example, a single large area heat film 332' is thermally connected (e.g., by gluing) to a large area metal sheet 333 (see Fig. 4B) which is thermally connected to each of the heat conductors 338. In this way, a single input 346 is connected to the heating film 332 to provide electrical power thereto, which is then converted to heat by the heating film 332' and evenly distributed to all of the heat conductors 338 under a single control scheme. One or more feedback sensors, such as thermocouples, may be thermally connected to the metal sheet 333 and electrically connected to the control board 334 as described above with the example of Fig. 3A.

As a variation to the example of Fig. 4A, a single large area heat film 332' is thermally connected (e.g., by gluing) to large area metal sheet 333 (see Fig. 4B) which is thermally connected to each of the heat conductors 338. The heat film 332' may alternatively be directly connected to power supply 330 via power cables 342 and 344 without any temperature control unit or temperature sensors. In this simplified arrangement, the temperature of the heat conductors 338 is adjusted by adjusting the power output of the power supply 330 and is a function of the resistance of the het film 332'.

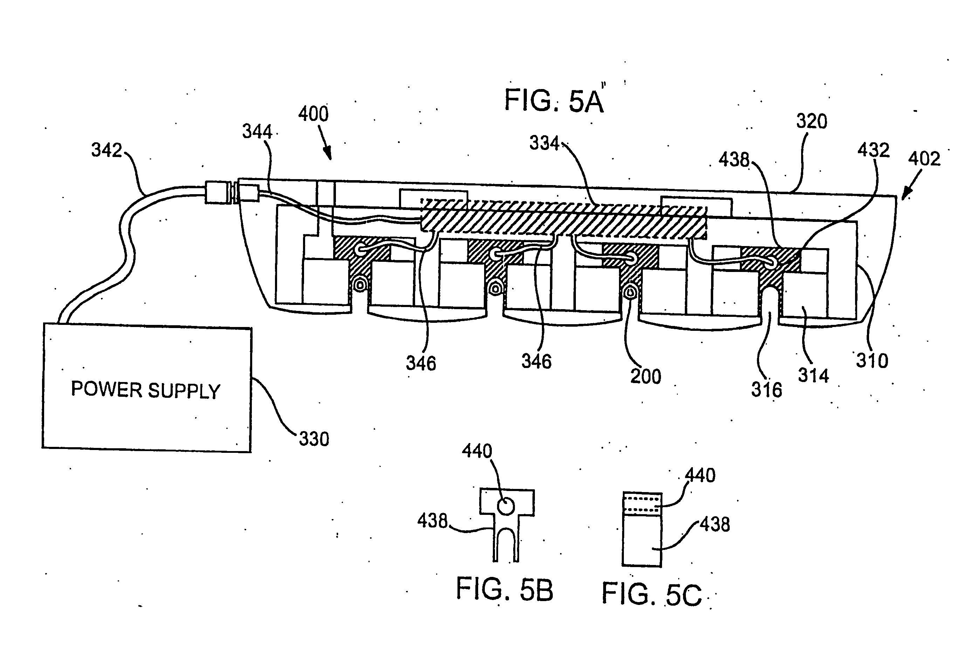

Another type of heating element that may be employed is a power resistance type heating element 432, as shown in Fig. 5A. In this example, a system 400 is provided with a separation unit 402 that employs power resistance type heating. A power resistance element 432 is thermally connected to a heat conductor 438 and is electrically connected to a power supply 330 via electronic control board 334. A power resistance element 432 and heat conductor 438 is provided for each gap 316 for receiving a column 200. This type of heating arrangement is generally larger and higher powered than the heating film element discussed above, and could be used in situations where higher temperature ranges may be required, or where faster heating response times would be of benefit. Generally, any resistance with a housing designed to be mounted to a metal profile heat sink, such that the heat produced in the resistance leaves the resistance housing from a defined flat surface will function as a power resistance element in this case. More specifically, power elements for use in this embodiment may be obtained from RS Components GmbH, Morfelden-WaHdorf, Germany: Vishay-Sfemice, 20W resistance in TO-220 housing, 0.1Ω to 10kΩ resistance, or Arcol, 10,15,25,50,100,150,200,300 or 600W type, embedded in an aluminum housing suitable for mounting to a metal profile heat sink, 22ohm to 50kohm resistance; or with a ceramic housing resistance of 1ohm to 10ohm, 4,7,11 or 17W type. One or more feedback sensors, such as one or more thermocouples, may be thermally connected to the metal sheet 333 and electrically connected to control board 334 similarly to that described with regard to Fig. 3A.

Fig. 5B is an isolated top view of a heat conductor 438 used in the embodiment of Fig. 5A. Heat conductor 438 may be formed from cast, anodized or other metal with good heat conducting properties (e.g., aluminum, stainless steel, copper or the like). A bore 440 or other opening configured to receive a power resistance element 432 is provided through each heat conductor 438 (as shown in phantom in the side view of Fig. 5C). A power resistance element is then inserted in opening 440 and thermally connected with the heat conductor as by gluing, for example (e.g., TBS thermal bonding system from Electrolube, Berkshire, England).

All of the preceding examples have been described as arrangements for providing a heating capability to the columns, such that the contents of the columns may be heated *in situ*, during processing. However, any of the foregoing arrangements may also be coupled with a cooling arrangement to give the resulting system the capability of cooling as well as heating, *in situ,* as will become more apparent in the description below.

The thermoelectric Peltier effect is the most direct way to utilize electricity to pump heat. Peltier elements may be used as heating/cooling elements 532 to provide both heating and cooling functions. Electric current forces one side of a Peltier element to approach a higher energy state, where heat is absorbed, thus providing a cooling effect in the vicinity of this side. The other side of the Peltier element is forced toward a lower energy state, where the energy is released which causes a heating effect in the vicinity thereof. The electric current can be reversed to cause the opposite effect on the respective sides, therefor the side facing the column or magnets can be used to either heat the column or cool it.

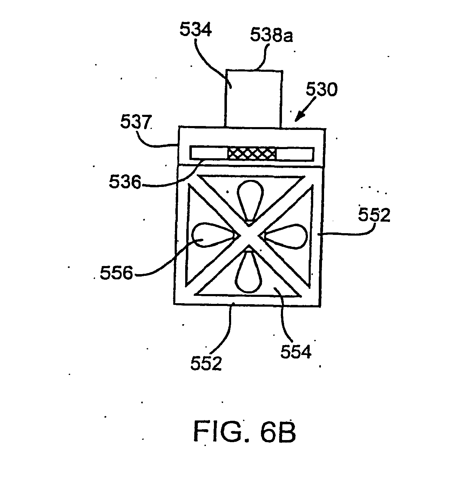

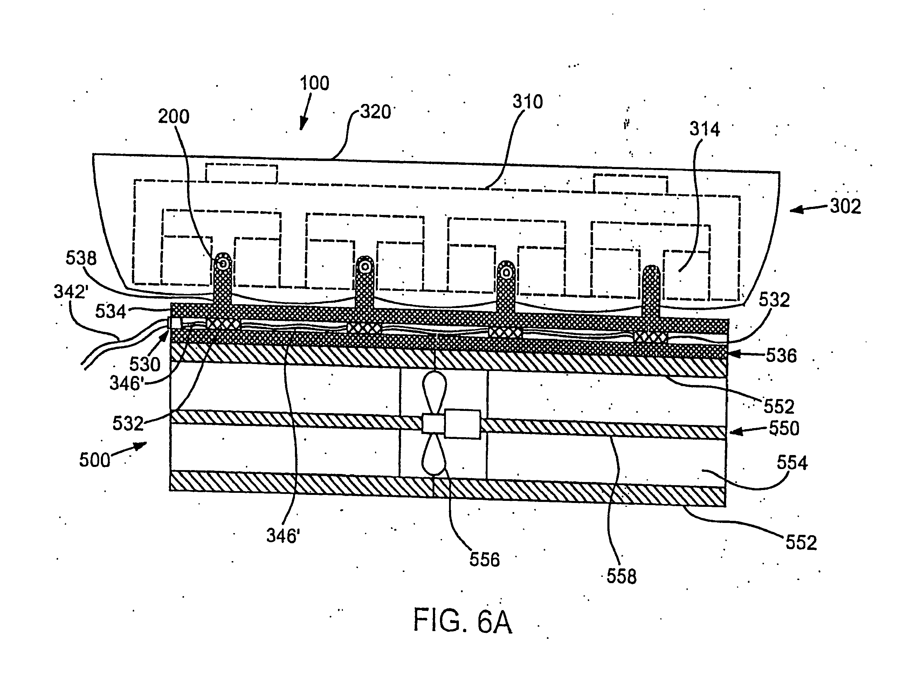

In the example shown in Fig. 6A, an external regulating unit 500 is provided which is useable with a standard HGMS system such as system 100 shown and described above with reference to Fig. 1. External regulating unit 500 includes Peltier elements 532 mounted within a heat conducting element and spaced so as to align a Peltier element 532 with each column 200 when the external regulating unit 500 interfaces with the separation unit 300. The heat conducting element may be made of aluminum, for example, or other metal with good heat conducting properties. The front and back 534 and 536, respectively, of the heat conducting element 530 are integrally joined by sides 537 to fully surround the Peltier elements 532 and to allow efficient heat transfer throughout the heat conducting element. The front 534, back 536 and sides 537 are preferably, but not necessarily all formed of the same material, usually aluminum.

The front 534 of the heat conducting element 530, i.e., that portion that is to interface with the separation unit 300, is provided with thermally conducting fingers 538 that are dimensioned and spaced to fit within the gaps 316 of the separation unit 300. The end of each finger is provided with a concave surface 538a which is adapted to abut and closely interface with a portion of the circumference of a column 200 to establish good heat transfer between the column and the finger. The width of each finger 538 is substantially the same as or only minimally less than the width of a gap 317, so that the finger substantially fills the remainder of the gap that is left after a column is inserted. The height of a finger should be at least as great as a height of the matrix and material to be processed within the column 200, for best results. The length of the fingers 538 are preferably only as long as will allow contact between ends 538a and columns 200, thereby positioning the front 534 of the heat conducting element 530 in close approximation with the separation unit 300. Although longer fingers could be used, they would be less efficient, as the resulting gap between the heat conducting element 530 and the separation unit 300 would allow for more heat losses between the two components. Shorter fingers would not allow contact between the ends of the fingers and the columns and would therefor not function acceptably.

A heating/cooling sink 550 is thermally connected to the heat conducting element to aid in the dissipation of heat (during cooling of the columns 200) or to collect and conduct heat to the Peltier elements 532 heat (during heating of the columns 200). The heating/cooling sink may be made of a good heat conducting metal, such as anodized aluminum, stainless steel, passivated copper (chrome/nickel plated), or the like for example, and should be formed to have a large surface area to thickness ratio, as known to those skilled in heat sink manufacture. In the example shown, the heating/cooling sink forms a large channel structure with relatively thin walls 552 and a large air space 554 formed there between. Optionally, a fan 556 may be provided to enhance the flow of air through the air space 554 for improved heat dissipation. One or more fans may be used and may be placed within the heat sink (as shown), or at an end thereof. The fan(s) may be powered by the same power supply that powers the Peltier elements 532, or by a separate power source, AC or DC. In the example shown, optional fan 556 is mounted on a support rod which is also composed of a good heat conducting metal, such as aluminum.

The heating cooling sink may also be optionally formed with fins (not shown) to increase the available surface area thereof. Heat sinks may also be provided with the other heating examples mentioned above, to increase the response time for cooling the magnets when heating energy is not being applied.

The Peltier elements 532 are electrically connected to a power supply 330 (not shown, but may be the same as those described previously) by a power cord 342' which connects the power supply with the external regulating unit 500 and by electrical conductors 346' which interconnect the Peltier elements 532. One or more feedback sensors, such as one or more thermocouples or the like, may be thermally connected to the fingers 538 and electrically connected to a control board (not shown) in a manner similar to that described with regard to Fig. 3A above.

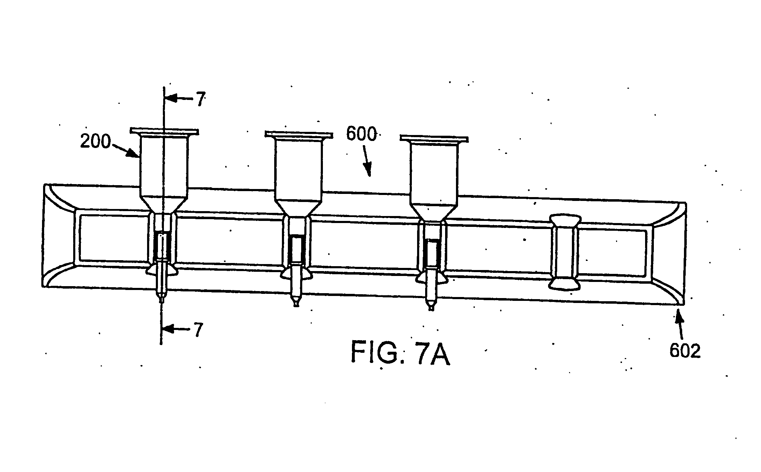

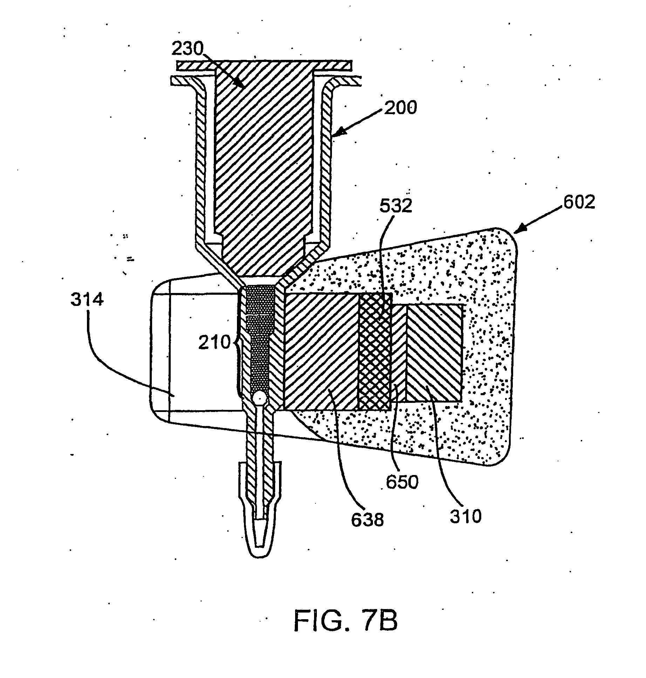

Peltier elements 532 may also be provided internally in a separation unit, as in separation unit 602 in the system 600 shown in Fig. 7A. In this configuration, a power line (not shown) form an external power supply like that discussed in previous embodiments, is electrically connected to each of the Peltier elements 532. A separate Peltier element is mounted behind each of the gaps into which columns 200 are received. A heat conductor 638, such as aluminum, for example is thermally connected with each Peltier element and is configured to contact and interface with a column 200 and magnets 314. A heat/cooling sink 650 is thermally connected to each Peltier element 532 opposite the side on which heat conductor 638 is thermally connected and may be thermally connected to yoke 310 to aid in heat dissipation. Alternatively, the yoke 310 may be directly thermally connected to each Peltier element 532 to serve as a heat/cooling sink therefor. Optionally, either arrangement may be further modified so as to thermally connect the yoke with a metallic stand or other ferromagnetic material (not shown) to increase the ability of the system to dissipate heat. One or more feedback sensors may be provided, and connected as described above with previous embodiments, so as to provide a feedback loop used in controlling the temperature.

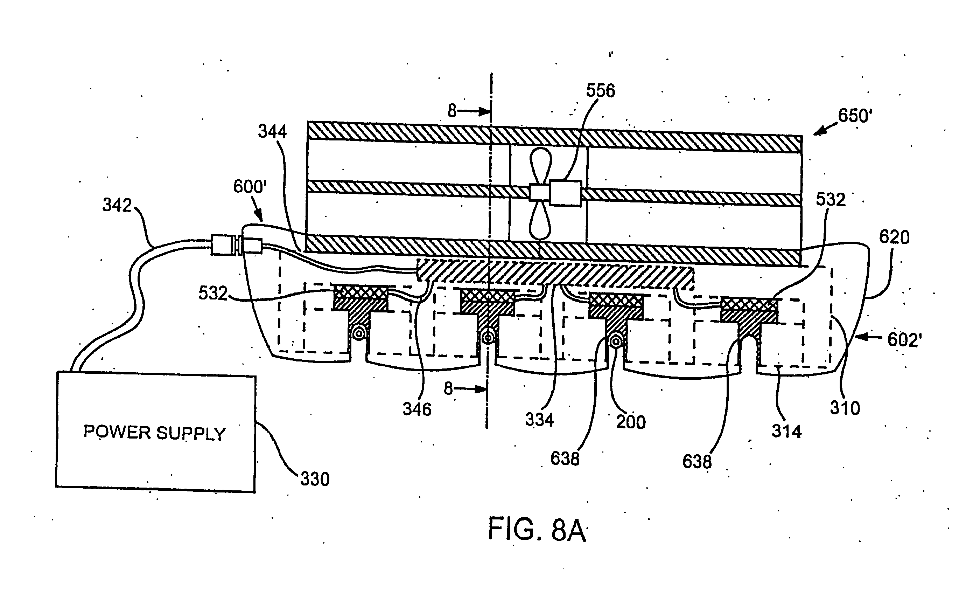

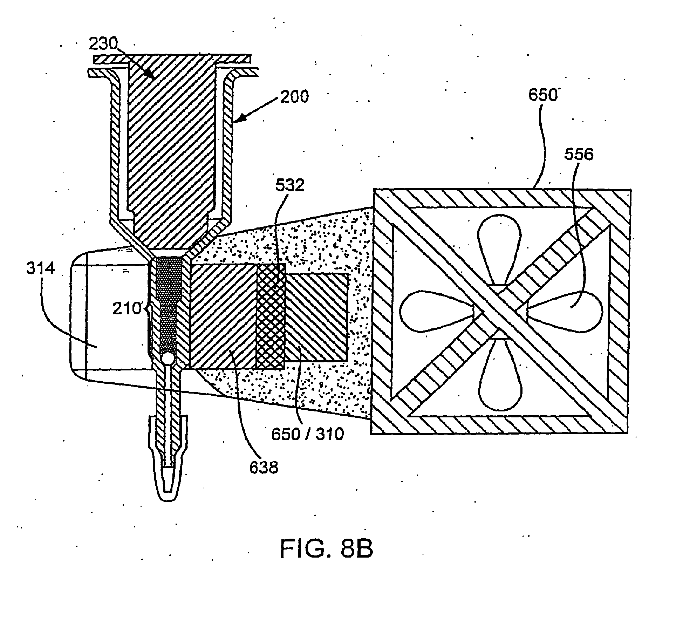

Figs. 8A-8B show a modification of an internal Peltier arrangement like that shown in Figs. 7A-7B with the difference being that in addition to thermally connecting the Peltier elements to the yoke 310 to function as a heat sink, the yoke 310 is further thermally connected to an external heat/cooling sink 650' being constructed with the properties described above with regard to heat/cooling sink 550. Again, the heat/cooling sink may optionally be provided with one or more fans 556 for increased heat dissipation capability. The housing 620 of separation 602' is essentially the same as housing 320 previously described, with the exception that an opening is provided in the back thereof to allow direct thermal contact and mounting of the heat/cooling sink 650' to the yoke 310. Optionally, the heat/cooling sink 650' and/or yoke may be further thermally connected to a metallic stand that supports the system (not shown) or other ferromagnetic material (not shown) to increase the ability of the system to dissipate heat.

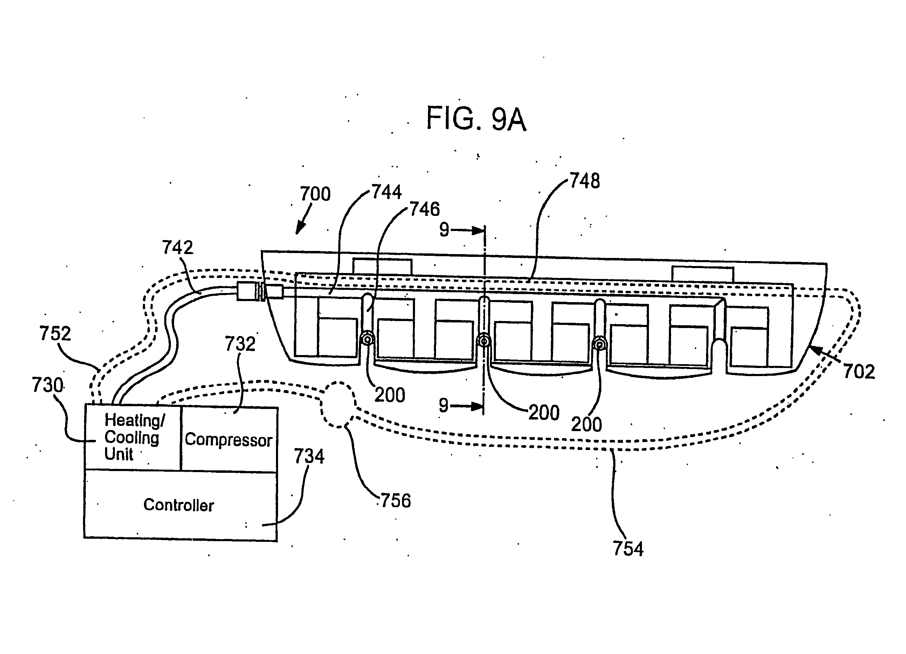

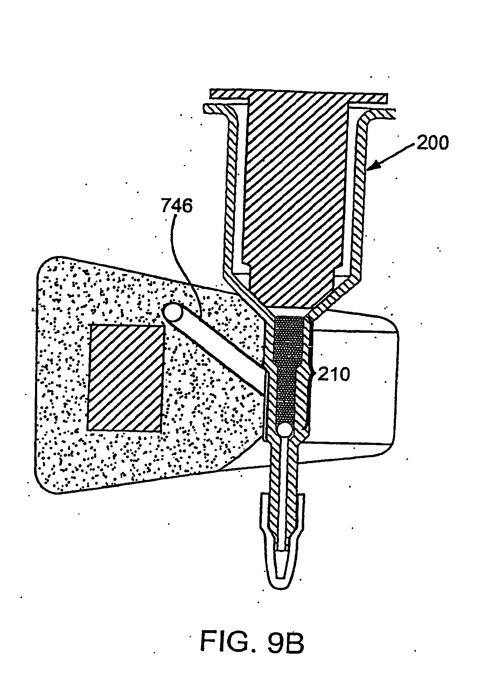

Turning to Fig. 9A, a partial sectional view of an HGMS system 700 with pneumatic heating and cooling is shown. A pneumatic tube 742 connects a heating/cooling unit 730 to the separation unit 702 via an air chuck which may be any of a number of pneumatic connectors known in the art. Pneumatic pipeline 744 runs substantially the length of separation unit 702 and connects the pneumatic tube 742 with pneumatic pipelines 746. Pipelines 746 deliver the pumped air to each of the individual separation columns 200 that are mounted in the separation unit 702. Pipelines 746 abut or very closely approximate the columns 200 at the location of the matrix 210 when in position in the separation unit 702, as shown in the sectional drawing of Fig. 9B. Water pipelines 748 may optionally be installed to pass through the yoke 310 to heat or cool the yoke 310 and the complete separation unit 702 in general, to minimize heat losses or gains to the pipelines 746 as they deliver their temperature controlled air to the columns 200 and to make a more uniform temperature throughout the system so as to stabilize the temperature control of the process(es) being conducted within the columns 200.

A compressor 732 located externally of the separation unit 702 develops compressed air which is fed into the heating/cooling unit 730 which may be a Peltier heating/cooling unit, for example. If used, the water through optional water pipelines 748 may also be heated or cooled bypassing the water through the heating/cooling unit 730, with water line 752 delivering water from the heating/cooling unit 730 to the pipelines 748, and pipeline 754 recycling water from the pipelines 748 to a pump 756 used to drive the circulation. Alternatively, the water lines may be pumped through a separate heat exchanger to control the temperature of the water therein. The separate heat exchanger may be a Peltier type, or a compression system or other know heat exchange design. Also, known cooling/heating fluids other that water may be passed through this optional system. Controller 734 may be manually set or automatically programmed to control the heating/cooling unit as to whether the inputted compressed air is to be heated or cooled and as well as to control the temperature that the compressed air is to be outputted at from the heating/cooling unit 730. A thermostatically controlled output valve (not shown) may be fitted on the output side of the heating/cooling unit to ensure that no airflow exits the heating/cooling unit until the air reaches a predetermined temperature. For example, a 1/8" 24V DC valve available from RS components, Morfelden-Walldorf, Germany, may be used. Controller 734 may also be configured to adjust the temperature at which the thermostatically controlled valve is to open, as well as to control the temperature of the water/liquid heating/cooling lines and the pump 756. Additionally, one or more thermocouples or other feedback mechanisms may be provided for controlling temperature at the site of the columns more precisely.

Although Figs. 9A and 9B show an example of an HGMS system which internally incorporates the pipelines for pneumatic heating/cooling of the columns 200, an external arrangement may be alternatively provided. Such an external arrangement would employ a heat conducting element like that described with regard to Fig. 6A above. Rather than employing Peltier elements in the heat conducting element, however, this example would include pneumatic pipelines through the heat conducting element and passing through the fingers to abut or closely approximate the columns 200 from the front side when the external heat conducting element is interfaced with the separation unit.

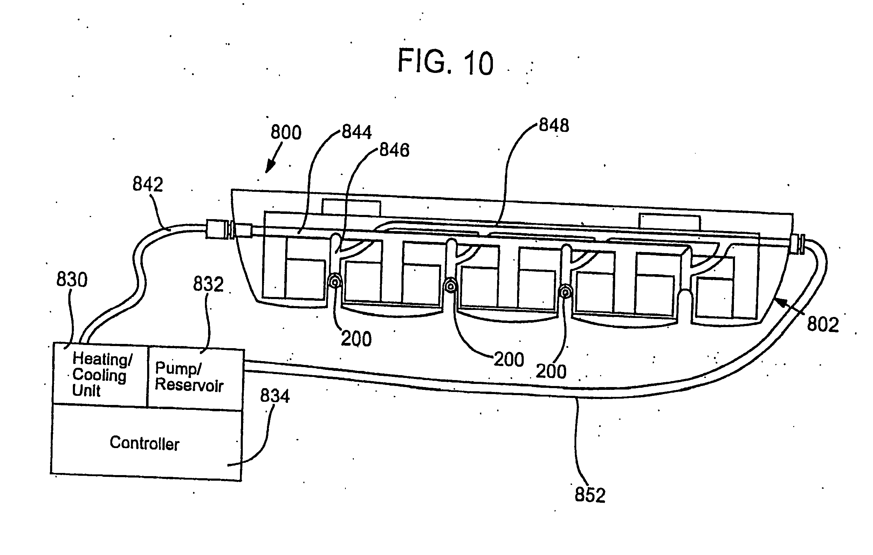

In Fig. 10 an HGMS system 800 employs a hydraulic heating/cooling arrangement. A hydraulic line 842 connects a heating/cooling unit 830 to the separation unit 802 via a hydraulic connector which may be any of a number of hydraulic connectors known in the art. Hydraulic pipeline 844 runs substantially the length of separation unit 802 and connects the hydraulic line 842 with hydraulic pipelines 846. Pipelines 846 carry temperature controlled fluid to each of the individual separation columns 200 that are mounted in the separation unit 702. Pipelines 846 abut or very closely approximate the columns 200 at the location of the matrix 210 when in position in the separation unit 802. Alternatively, the pipelines 846 may include an arrangement of thin fins (not shown) adjacent the columns, formed of a good heat conducting material, such as aluminum or copper, for example, configured for fluid flow therethrough, much like a radiator. In either configuration, temperature regulated fluid is circulated past the columns 200, where the columns are warmed or cooled, as appropriate. Circulating fluid is returned to a return hydraulic pipeline 848 which delivers the fluid out of the separation unit 802 to be returned to a reservoir 832 by return hydraulic line 852.

The reservoir 832 includes a pump which drives the circulation of the hydraulic fluid into a heat exchanger 830 which may be a Peltier heating/cooling unit, for example, and through separation unit 802 as described. A controller 834 may be provided externally, as shown, or internally of the unit 800, similar to the control boards discussed previously.

Controller 834 may be manually set or automatically programmed to control the heating/cooling unit and pump to ultimately control the temperature of the separation columns, by regulating the fluid temperature and the rate at which it is pumped through the system. The controller 834 receives inputs from feedback sensors located at the site of the columns (not shown) similar to the thermocouples discussed above, which are representative of the temperature measured by each thermocouple. The controller 834 includes a microprocessor which converts the inputs from the feedback sensors to temperature readings, compares the temperature readings to temperature settings which are either manually inputted into the controller by an operator or programmed in, and determines whether cooling, heating or stasis of each individual source is required. Although the example shown in Fig. 10 is a batch processor, where all of the columns are either heated, cooled, or maintained in stasis together, it would be within the skill of those of ordinary skill in the art to form independent feedback loops to each column with independent control over heating cooling and stasis.

Generally, in an arrangement where a heating/cooling source and a thermocouple are provided for each respective column, the temperature of each column may be individually controlled and regulated, such as in the examples shown and described in reference to Figs. 3A and 5A-10A. Such control may be advantageously employed to provded more precise adjustments of column temperatures (e.g., for very precisely controlled batch processing) or to expose different columns to different temperatures at the same time (e.g., differing temperature profiles for various columns held on the same system).

The appropriate action is taken by controlling electrically controlled throttled valves (not shown) or equivalent flow control mechanism in the line or lines inputting or exiting a location adjacent a column to be controlled, to change the amount of flow of the heating/cooling fluid which is circulated through one or more of the pipelines /heating elements 846. Additionally, the controller 834 may be configured to reverse the cycle of flow through the pipelines/heating elements 846 when it is determined that a change from heating to cooling or *vice versa* is required. Program routines may be stored in the controller 834 (or control board, as the case may be) for automatically controlling the temperature of the pipelines/heating elements 846 for various heating/cooling processes and cycles of treatment described below.

Although Fig. 10 shows an example of an HGMS system which internally incorporates the pipelines for hydraulic heating/cooling of the columns 200, an external arrangement may be alternatively provided. Such an external arrangement would employ a heat conducting element like that described with regard to Fig. 6A above. Rather than employing Peltier elements in the heat conducting element, however, this example would include hydraulic pipelines through the heat conducting element and circulating through the fingers to abut or closely approximate the columns 200 from the front side when the external heat conducting element is interfaced with the separation unit. Return lines recirculate the flow of hydraulic fluid from the fingers, out of the external unit to a pump, in a fashion similar to that described above with regard to the internal arrangement of Fig. 10. Standard Direct-to-Liquid heating /cooling elements are available, e.g., from Telemeter Electronic GmbH, Donauwoerth, Germany (e.g., DL-046-12-00, 37 W power input).

Further, both internal and external embodiments may be configured with independent hydraulic lines to each slot 316 so that each station/column may be independently heat/cool controlled. In any arrangement, although water or an aqueous solution is currently preferred for the cooling liquid, other fluids, such as chloro-fluoro carbons, or other known fluids used for heat transfer may be used.

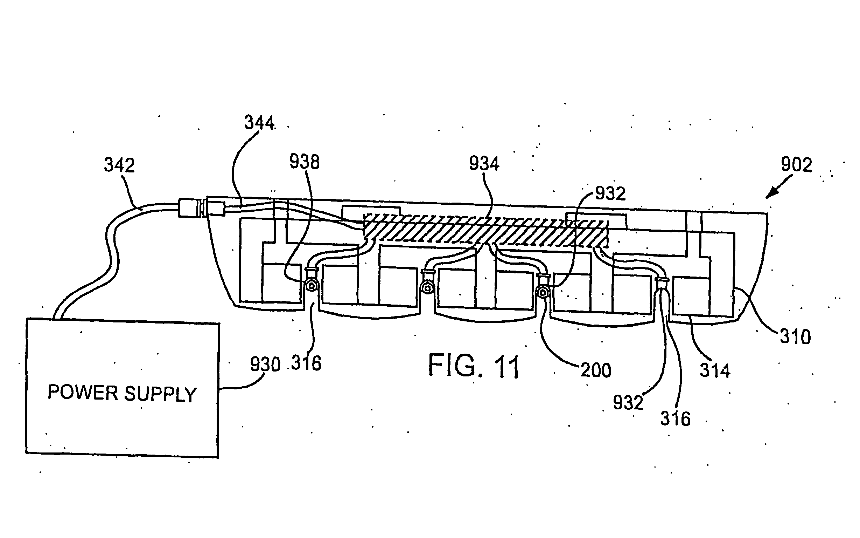

Fig. 11 is another example of an arrangement for heating the columns 200 in a separation unit 902. In this arrangement radiation type emitting element 932, such as an infrared LED (light emitting diode, examples of which are TO39 GaA1As, type OD50L, RS Components, Mörfelden- Walldorf, Germany), for example, is provided in a each location or gap 316 in the separation unit which will receive a separation column 200, so as to abut or closely approximate the separation column 200 in the vicinity of the matrix 210, when the column is positioned in the separation unit 902. Each emitting element 932 may be mounted to a respective column holder 938, to closely interface with a back side of each respective column 200, as shown in Fig. 11. For example, the column holder 938 may be provided with a concave cylindrical end adapted to mate with the column 200. Thus, radiation from the emitters 932 is provided directly to the columns.

An electronic control board 934 is provided in the unit 902 and is electrically connected to each of the emitting elements or sources 932 and an external power supply 930, which may be of the type used in the example described with regard to Fig. 3A above.

A power cord 342 connecting the power supply 930 to the unit 902 may be releasably coupled to a power cord 344 leading to the electronic control board 934, by any of a number of well known jack couplers having a male and a female component. The emitters 932 are independently connected to the electronic board 934 by respective electrical conductors 346 to allow independent temperature control over each source 932 by the control board 934.

Feedback sensors 948, such as infrared detectors or thermocouples, for example may be provided near each emitting element 932, on the surfaces of the column holder elements 938 that contact the columns 200, for example, and are electrically connected to the control board 934 to feed back signals which are representative of the temperature measured by each feedback sensor 948. The control board 934 includes a microprocessor which receives inputs from the thermocouples and converts them to temperature readings, compares the temperatures to temperature settings which are either manually inputted to the control board by an operator, or programmed in, and determines whether heating, cooling or stasis of each individual source is required. The appropriate action is taken by controlling the amount of power inputted from the power supply 930. Programmed routines may be stored in the control board for automatically controlling the temperature of each heating/cooling source for various processes and cycles of treatment as described below.

Although Fig. 11 shows an example of an HGMS system which internally incorporates the emitters and control board, an external arrangement may be alternatively provided. Such an external arrangement would employ a heat conducting element like that described with regard to Fig. 6A above. Rather than employing Peltier elements in the heat conducting element, however, this example would include emitting elements 932 at the ends of the fingers of the heat conducting element to abut or closely approximate the columns 200 from the front side when the external heat conducting element is interfaced with the separation unit.

Heating sources might be infrared LED or an infrared heating lamp (e.g. 100W Philips by RS Components, Mörfelden-Walldorf, Germany). In an external arrangement the LED or heating lamp could be focused on the columns 200 (by slots, mirrors, glass fiber light guides or the like) with one light source for all columns, or an individual light source being provided for each respective column and incorporated into the respective finger interfacing therewith.

Control board 934 could either be incorporated on the external arrangement, or electrically connected thereto from a separate location. A feedback mechanism, such as one or more infrared detectors or thermocouples, may also be incorporated into the fingers.

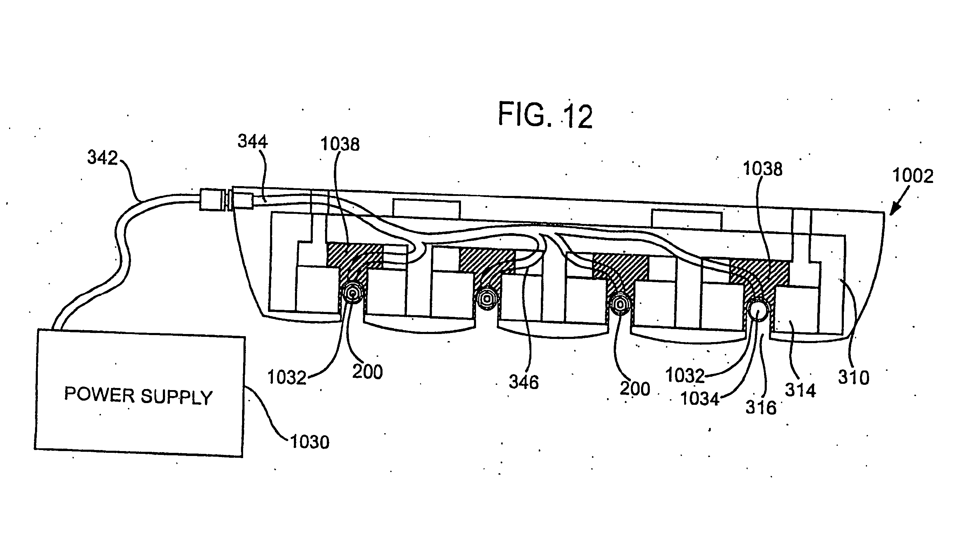

Yet another arrangement for heating columns 200 in a separation unit 1002 is shown in Fig. 12, in which spools 1032 of wound wire are mounted in the slots 316 of the separation unit. Each spool 1032 is substantially annular and has a central opening 1034 dimensioned to receive a column 200 therein. Each spool 1032 is electrically connected to a power source 1030 via electrical connection lines 342, 344 and 346, as shown. Power source 1030 is configured to apply alternating current to the spools 1032. When a column 200 is present in a spool and alternating current is applied, the alternating current through the wires of the spool induces heat in the electrically conducting components (e.g., iron spheres) of the matrix 210.

Although Fig. 12 shows an example of an HGMS system which internally incorporates the spools 1032, an external arrangement may be alternatively provided. Such an external arrangement would employ spools 1032 in the fingers of an external heat conducting element, having structural properties like the other external embodiments described above. The fingers, when inserted into slots 316 would then be positioned to receive columns 200 in the same the same manner as described above with the embodiment of Fig. 12.

## METHODS OF MODIFYING A BIOMOLECULE

The present invention provides methods for modification of magnetically immobilized biomolecules using a magnetic separation device, such as a magnetic separation device and/or system of the invention. The methods generally comprise immobilizing a biomolecule in a device and/or system of the present invention, and modifying the immobilized biomolecule. In some embodiments, a biomolecule is magnetically labeled before being applied to the magnetic separation device. In other embodiments, a biomolecule is associated with a second member which is magnetically labeled, such that, through the association with the magnetically labeled second member, the biomolecule becomes immobilized on the column.

In many embodiments, one or more modification steps are conducted under temperature-controlled conditions. Temperature-controlled conditions are generally achieved by adjusting the temperature of a device and/or system of the invention. In some embodiments, the method further comprises the step of separating a reaction product from an immobilized biomolecule. In other embodiments, the method further comprises the step of eluting the modified biomolecule from the column. In still other embodiments, the method comprises conducting two or more modification steps. In all embodiments, additional steps, such as one or more intervening elution and/or washing steps and/or inactivation steps, may be included. Thus, the invention further provides methods of isolating a modified biomolecule, comprising modifying a biomolecule as described herein, and isolating the modified biomolecule.

Furthermore, in many embodiments of interest, modification of a selected biomolecule results in generation of at least a second biomolecules. In some of these embodiments, the second biomolecule is a modified biomolecule, e.g., a modification of a first, magnetically immobilized biomolecule. In other embodiments, the second biomolecule is a newly synthesized biomolecule, e.g., a newly synthesized biomolecule using a magnetically immobilized biomolecule as the source of information from which to synthesize the second biomolecule. In some embodiments, the second biomolecule is eluted. In other embodiments, the second biomolecule is captured by a second binding moiety that is immobilized in the separation device. In some of these embodiments, the captured second biomolecule is further modified, or is purified without modification.

In some embodiments, the invention provides a method for modifying a biomolecule. The method generally involves a) immobilizing a biomolecule bound to a magnetic particle on a magnetic separation apparatus by applying a magnetic field to a magnetizable matrix in the column; and b) modifying the immobilized biomolecule, wherein the modification is conducted at a temperature that is suitable for modification. The temperature suitable for modification is attained by adjusting the temperature of the column, e.g., using a device of the invention. In some of these embodiments, the modification is an enzymatic modification with at least a first enzyme, and the apparatus is maintained for a first period of time at a first temperature at which the first enzyme exhibits at least 10% of its maximal activity. In some embodiments, the method further includes a step of c) modifying the immobilized biomolecule with a second enzyme, wherein the apparatus is maintained for a second period of time at a second temperature at which the second enzyme exhibits at least 10% of its maximal activity. In many embodiments, the method further includes the step of eluting the modified biomolecule from the column.

In other embodiments, the invention provides a method of synthesizing a nucleic acid molecule. The method generally involves immobilizing a biomolecule bound to a magnetic particle on a magnetic separation apparatus by applying a magnetic field to a magnetizable matrix in the column, wherein the immobilized biomolecule comprises a polynucleotide and wherein the magnetic particle contains bound thereto an oligonucleotide that is complementary to a portion of the immobilized biomolecule and that serves as a primer for synthesis of a nucleic acid; contacting the immobilized polynucleotide with an enzyme that can synthesize a nucleic acid molecule, in the presence of deoxynucleotides, wherein the apparatus is maintained for a period of time at a temperature at which the enzyme exhibits at least 10% of its maximal activity; and synthesizing a nucleic acid molecule, using the immobilized polynucleotide as a template. In some embodiments, at least one deoxynucleotide comprises a detectable label, and the synthesized nucleic acid molecule includes the at least one detectably labeled deoxynucleotide.

In other embodiments, the invention provides a method of synthesizing a nucleic acid molecule. The methods generally involve immobilizing a biomolecule bound to a magnetic particle on a magnetic separation apparatus by applying a magnetic field to a magnetizable matrix in the column, wherein the immobilized biomolecule comprises a polynucleotide; contacting the immobilized polynucleotide with a first oligonucleotide primer and an enzyme that can synthesize a nucleic acid molecule, in the presence of deoxynucleotides, wherein the apparatus is maintained for a period of time at a temperature at which the enzyme exhibits at least 10% of its maximal activity; and synthesizing a nucleic acid molecule, using the immobilized polynucleotide as a template.

In other embodiments, the invention provides a method of synthesizing a nucleic acid molecule. The method generally comprises immobilizing a biomolecule bound to a magnetic particle on a magnetic separation apparatus by applying a magnetic field to a magnetizable matrix in the column, wherein the immobilized biomolecule comprises a polynucleotide comprising a poly(A) tract and the magnetic particle is bound to an oligo-dT molecule of from about 6 nucleotides to about 30 nucleotides; contacting the immobilized polynucleotide with an enzyme that can synthesize a nucleic acid molecule, in the presence of deoxynucleotides, wherein the apparatus is maintained for a period of time at a temperature at which the enzyme exhibits at least 10% of its maximal activity; and synthesizing a nucleic acid molecule, using the immobilized polynucleotide as a template.

Biomolecules

Any of a variety of biomolecules are suitable for modification using a method of the invention, including, but not limited to, polypeptides; polynucleotides; lipids; polysaccharides; lipoproteins; glycoproteins; peptide nucleic acids (PNA); locked nucleic acid molecules (LNA); and derivatives and analogs of any of the foregoing.

A biomolecule may comprise two or more moieties belonging to different categories of biological molecule (e.g., polypeptide, polynucleotide, saccharide, and lipid), e.g., a biomolecule may comprise a polypeptide moiety and a polynucleotide moiety (e.g., a peptide nucleic acid). Furthermore, a biomolecule may comprise two or more moieties, each belonging to different chemical classes of compounds. Examples of such biomolecules are conjugates.

Conjugates of nucleic acid molecules and non-nucleic acid molecules, and methods for making same, are known in the art and described in, for example, WO 98/16427, WO 98/55495, WO 00/21556, each of which is incorporated by reference for their teachings relating to conjugates. Further teachings relating to nucleic acid conjugates may be found in S.L. Beaucage, ed. (1999) Current Protocols in Nucleic Acid Chemistry, John Wiley & Sons; and Kisakurek et al., eds. (2000) Frontiers in Nucleic Acid Chemistry, John Wiley & Sons. Where the non-nucleic acid moiety is a peptide, the peptide portion of the conjugate can be attached to the nucleic acid molecule through an amine, thiol, or carboxyl group in the peptide. If the peptide antigen contains a suitable reactive group (e.g., an N-hydroxysuccinimide ester) an immunomodulatory nucleic acid molecule can be reacted directly with an epsilon amino group of a lysine residue. The peptide portion of the conjugate can be attached to the 3' end of the nucleic acid molecule through solid support chemistry. For example, the nucleic acid molecule portion can be added to a polypeptide portion that has been pre-synthesized on a solid support (see, *e.g.,* Haralambidis et al. (1990) Nucl. Acid Res. 18:493-499; Haralambidis et al. (1990) Nucl. Acid Res. 18:501-505). Alternatively, the nucleic acid molecule can be synthesized such that it is connected to a solid support through a cleavable linker extending from the 3' end. Upon chemical cleavage of the nucleic acid molecule from the support, a terminal thiol group, or a terminal amino group, is left at the 3' end of the nucleic acid molecule (*e.g.*, Zuckermann et al. (1987) Nucl. Acids Res. 15:5305-5321; Nelson et al. (1989) Nucl. Acids. Res. 17:1781-1794). Conjugation of an amino-modified nucleic acid molecule to amino groups of the peptide can be performed as described (see, *e.g.,* Benoit et al. (1987) Neuromethods 6:43-72). Conjugation of a thiol-modified nucleic acid molecule to carboxyl groups of a peptide antigen can be performed as described (see, *e.g.,* Sinah et al. (1991) Oligonucleotide Analogues: A Practical Approach, IRL Press). Coupling of a nucleic acid molecule carrying an appended maleimide to the thiol side chain of a cysteine residue of a peptide can also be performed (see, *e.g.,* Tung et al. (1991) Bioconj. Chem.I 2:464-465).

The peptide portion of a conjugate can be attached to the 5- end of a nucleic acid molecule through an amine, thiol, or carboxyl group that has been incorporated into the nucleic acid molecule during its synthesis (see, *e.g.,* Agrawal et al. (1986) Nucleic Acids Res. 14:6227-6245; Bischoff et al. (1987) Anal. Biochem. 164:336-344; and U.S. Patent Nos. 4,849,513; 5,015,733; 5,118,800; and 5,118,802).

The linkage of a nucleic acid molecule to a lipid can be formed using standard known methods. These methods include, but are not limited to, the synthesis of oligonucleotide-phospholipid conjugates, oligonucleotide-fatty acid conjugates, and oligonucleotide-sterol conjugates (see, *e.g.,* Yanagawa et al. (1988) Nucleic Acids Symp. Ser. 19:189-192; Grabarek et al. (1990) Anal. Biochem. 185:131-135; and Boujrad et al. (1993) Proc. Natl. Acad. Sci. USA 90:5728-5731).

Linkage of a nucleic acid molecule to an oligosaccharide or polysaccharide can be performed using standard known methods, including, but not limited to, the method described in O'Shannessy et al. (1985) J. Applied Biochem. 7:347-355.

A conjugate can be formed through covalent bonds, as described above. A conjugate can also be formed through non-covalent interactions, such as ionic bonds, hydrophobic interactions, hydrogen bonds, and/or van der Waals attractions.

Where the non-nucleic acid moiety is a polypeptide, the polypeptide may be conjugated directly or indirectly to a nucleic acid molecule, *e.g.,* conjugated to the nucleic acid molecule via a linker molecule. A wide variety of linker molecules are known in the art and can be used in the conjugates. The linkage from the peptide to the nucleic acid molecule may be through a peptide reactive side chain, or the N- or C-terminus of the peptide. Linkage from the nucleic acid molecule to the peptide may be at either the 3' or 5' terminus, or internal. A linker may be an organic, inorganic, or semi-organic molecule, and may be a polymer of an organic molecule, an inorganic molecule, or a co-polymer comprising both inorganic and organic molecules. A linker may also be a bead derivatized to contain appropriate groups for attachment of a nucleic acid molecule and an antigen. A wide variety of beads, including biodegradable beads, as well as methods of linking molecules to beads, are well known to those skilled in the art.

If present, the linker molecules are generally of sufficient length to permit oligonucleotides and/or polynucleotides and a linked polypeptide to allow some flexible movement between the nucleic acid molecule and the polypeptide. The linker molecules are generally about 6-50 atoms long. The linker molecules may also be, for example, aryl acetylene, ethylene glycol oligomers containing 2-10 monomer units, diamines, diacids, amino acids, or combinations thereof. Other linker molecules which can bind to oligonucleotides may be used in light of this disclosure.

A population of biomolecules to be modified may be homogeneous or substantially homogeneous with respect to one moiety, but heterogeneous with respect to a second moiety, where the first and second (or additional) moieties belong to different chemical classes. For example, a population of biomolecules may all comprise the same polynucleotide moiety (e.g., an identical sequence of nucleotides), but may comprise a peptide moiety that differs among the members of the population of biomolecules.

Still further, a population of biomolecules to be modified may be homogeneous or substantially homogeneous with respect to one moiety, but heterogeneous with respect to a second moiety, where the first and second (or additional) moieties belong to the same chemical classes. For example, a population of biomolecules may comprise a sequence of polynucleotides or a sequence of amino acids that is identical in all members of a population, and a sequence of polynucleotides or a sequence of amino acids that differs among members of the population. For example, all members of a population of biomolecules may comprise a polynucleotide sequence encoding a particular protein domain; and a polynucleotide having a sequence that is divergent among the members of the population.

Preparation ofbiomolecules

A biomolecule may be isolated from a biological source (e.g., an animal, a plant, a virus, a bacterium, a protozoan, and the like); may be synthesized using conventional methods; may be recombinant; or may be isolated from a biological entity and modified synthetically or enzymatically. In some embodiments, a biomolecule or population of biomolecules is isolated from a heterogeneous mixture before being applied to a magnetic separation device. In other embodiments, a biomolecule is modified prior to being applied to a magnetic separation device.

In some embodiments, a sample being applied to a separation device is enriched for a biomolecule that is to be modified, as compared to the environment in which is it naturally found, or compared to a starting sample such as a mixture comprising a synthesized biomolecule, or a biomolecule isolated from a biological source, then modified. As such, a biomolecule may be purified, where by purified is meant that the biomolecule is present in a composition that is substantially free of other components, e.g., other biomolecules, whereby substantially free is meant that less than 90 %, usually less than 60 % and more usually less than 50 % of the composition is made up of components (e.g., other biomolecules) other than the biomolecule to be modified.

In certain embodiments of interest, e.g., when the biomolecule of interest is isolated from a biological entity, a biomolecule is present in a composition that is substantially free of the constituents that are present in its naturally occurring environment. For example, a composition comprising a biomolecule will be substantially, if not completely, free of those other biological constituents, such as proteins, carbohydrates, lipids, etc., with which it is present in its natural environment. As such, biomolecule compositions of these embodiments will necessarily differ from those that are prepared by purifying the protein from a naturally occurring source, where at least trace amounts of the protein's natural environment constituents will still be present in the composition prepared from the naturally occurring source.

A biomolecule may also be present as an isolate, by which is meant that the biomolecule is substantially free of other naturally occurring and/or synthetic biological molecules, particularly other biological molecules that are unrelated in structure, (e.g., where the biomolecule is a polynucleotide, biological molecules unrelated in structure include polysaccharides, oligosaccharides, proteins and fragments thereof), and the like, where substantially free in this instance means that less than 70 %, usually less than 60% and more usually less than 50 %, less than 40%, less than 30%, less than about 25%, less than about 20%, less than about 15%, less than about 10%, less than about 5%, or less than about 2% of the composition containing the isolated biomolecule is a biomolecule other than the biomolecule being isolated. In some embodiments, a modified biomolecule is isolated from other biomolecules unrelated in structure to the modified biomolecule and from other biomolecules that are related in structure but that are not modified.

In certain embodiments, the biomolecule is present in substantially pure form, whereby substantially pure form is meant at least 95%, usually at least 97% and more usually at least 99% pure. In some embodiments, a population of biomolecules (which may be a heterogenous population, e.g., a heterogeneous population of cDNA molecules; a heterogenous population of polypeptides) is isolated from other biomolecules. An isolated population ofbiomolecules is substantially free of other biomolecules, e.g., those biomolecules not containing the modification of interest, or other biomolecules unrelated in structure, and in many instances is substantially pure.

Polypeptides can be isolated from a biological source, can be produced synthetically, or can be produced recombinantly, i.e., a polynucleotide comprising a coding region encoding the polypeptide can be inserted into an expression vector, and the coding region transcribed and translated.

Polypeptides can be isolated from biological sources, using standard methods of protein purification known in the art, e.g., affinity chromatography, ion-exchange chromatography, hydrophobic interaction chromatography, size exclusion chromatography, salt precipitation, or a combination of two or more of the foregoing.

One may employ solid phase peptide synthesis techniques, where such techniques are known to those of skill in the art. *See* Jones, The Chemical Synthesis of Peptides (Clarendon Press, Oxford)(1994). Generally, in such methods a peptide is produced through the sequential additional of activated monomeric units to a solid phase bound growing peptide chain.

For expression, an expression cassette may be employed. The expression vector will provide a transcriptional and translational initiation region, which may be inducible or constitutive, where the coding region is operably linked under the transcriptional control of the transcriptional initiation region, and a transcriptional and translational termination region. These control regions may be native to the subject gene, or may be derived from exogenous sources.

Expression vectors generally have convenient restriction sites located near the promoter sequence to provide for the insertion of nucleic acid sequences encoding heterologous proteins. A selectable marker operative in the expression host may be present. Expression vectors may be used for the production of fusion proteins, where the exogenous fusion peptide provides additional functionality, i.e. increased protein synthesis, stability, protein solubility, cell membrane permeability, reactivity with particular ligands, reactivity with defined antisera, an enzyme marker, e.g. β-galactosidase, etc.

Expression cassettes may be prepared comprising a transcription initiation region, the gene or fragment thereof, and a transcriptional termination region. The polypeptides may be expressed in prokaryotes or eukaryotes in accordance with conventional ways, depending upon the purpose for expression. For large scale production of the protein, a unicellular organism, such as *E. coli, B. subtilis, S. cerevisiae,* insect cells in combination with baculovirus or non-viral vectors, or cells of a higher organism such as vertebrates, such as mammals, e.g. COS 7 cells, may be used as the expression host cells. In some situations, it is desirable to express the gene in eukaryotic cells, where the protein will benefit from native folding and post-translational modifications. Small peptides can also be synthesized in the laboratory. Polypeptides that are subsets of the complete amino acid sequence may be used to identify and investigate parts of the protein important for function, or to raise antibodies directed against these regions.

With the availability of the protein or fragments thereof in large amounts, by employing an expression host, the protein may be isolated and purified in accordance with conventional ways. A lysate may be prepared of the expression host and the lysate purified using HPLC, exclusion chromatography, gel electrophoresis, affinity chromatography, or other purification technique.

Polynucleotides can be prepared in a number of different ways. For example, polynucleotides can be prepared using standard isolation techniques known in the art. See, e.g., Short Protocols in Molecular Biology, (1999) F. Ausubel, et al., eds., Wiley & Sons; Sambrook, Maniatis, and Fritsch, (1989) Molecular cloning: A laboratory manual, Cold Spring Harbor Laboratory, Cold Spring Harbor, N.Y.; and Ausubel, F. M., et al., eds.. (1995) Current Protocols in Molecular Biology John Wiley & Sons, Inc., New York. Alternatively, the nucleic acid molecule may be synthesized using solid phase synthesis techniques, as are known in the art. Oligonucleotide synthesis is also described in Edge, et al., (1981) Nature 292:756; Duckworth et al., (1981) Nucleic Acids Res 9:1691 and Beaucage, et al., (1981) Tet. Letts 22: 1859.

Magnetic labeling

A biomolecule to be modified is first bound, directly or indirectly, to a magnetic particle. Methods for magnetically labeling a biomolecule are known in the art; any known method can be used. For example, U.S. Patent No. 6,020,210 describes methods for preparation of magnetic particles, and attachment of biomolecules thereto. A first member of a specific binding pair can be associated with a magnetic particle, wherein the biomolecule to be modified comprises a moiety that binds to the member of the specific binding pair.

Examples of members of specific binding pairs that can be attached to a magnetic particle include, but are not limited to, oligo dT (for binding to nucleic acid molecules comprising, e.g., a poly-A tract at the 3' end); oligonucleotides having a specific nucleotide sequence (for binding to nucleic acid molecules comprising a complementary nucleotide sequence); avidin (e.g., streptavidin) (for binding to a biotinylated biomolecule); an antigen-binding polypeptide, e.g., an immunoglobulin (Ig) or epitope-binding fragment thereof (for binding to a biomolecule comprising an epitope recognized by the Ig); polynucleotide binding proteins (for binding to a polynucleotide), e.g., a transcription factor, a translation factor, and the like; Ni or Co chelate (to immobilize poly-histidine-tagged proteins); receptor-ligand systems, or other specific protein-protein interacting pairs; aptamers (e.g., nucleic acid ligands for three-dimensional molecular targets); lectins (for binding glycoproteins); lipids and phospholipids (binding to lipid-binding proteins), e.g., phosphatidyl serine and annexin V. Those skilled in the art will recognize other members of specific binding pairs that may be attached to a magnetic particle.

A biomolecule can also be coupled (covalently or non-covalently) to a magnetic particle by direct chemical conjugation or by physical association. Such methods are well known in the art. Biochemical conjugations are described in, e.g., "Bioconjugate Techniques" Greg T. Hermanson, Academic Press. Non-covalent interactions, such as ionic bonds, hydrophobic interactions, hydrogen bonds, and/or van der Waals attractions can also be used to couple a biomolecule with a magnetic particle. For example, standard non-covalent interactions used to bind biomolecules to chromatographic matrices can be used. One non-limiting example of such a non-covalent interaction that can be used to bind a biomolecule to a magnetic particle are DNA binding to silica in the presence of chaotropic salts. Those skilled in the art are aware of other such non-covalent binding and conditions for achieving same. See, e.g., Molecular Cloning, Sambrook and Russell, Cold Spring Harbor Laboratory Press.

Magnetic field

Once a magnetically labeled biomolecule is applied to the separation device, a magnetic field is applied. Depending on the strength of the applied magnetic field, biomolecules can be fixed in place, or can be in a suspension. The suspension can be localized, e.g., in certain high magnetic field or gradient areas of the matrix; or throughout the entire void volume of the separation device.

In general, the applied magnetic field is in a range of from about 0.1 to about 1.5 Tesla, from about 0.2 to about 0.8 Tesla. In some embodiments, the magnetic field is reduced to zero.

In some embodiments, the magnetically labeled biomolecule is immobilized in suspension. The strength of the magnetic field that is applied to the separation device can be adjusted to provide for the formation of a suspension of the magnetic particles with which the biomolecules are associated. Keeping the biomolecules in suspension is advantageous for some applications, where homogeneous modification of the biomolecules is desired.

Modifications

A biomolecule may be modified before being applied to a separation device and/or may be modified after being applied to a separation device and immobilized therein. A biomolecule may be subjected to more than one modification, before and/or after being applied to the separation device. As used herein, the term "modification" includes altering the structure of the magnetically immobilized biomolecule; binding another molecule to the magnetically immobilized biomolecule (e.g., via a nucleic acid/nucleic acid, a protein/nucleic acid, or a protein/protein interaction, and the like); and synthesizing a new biomolecule using the magnetically immobilized biomolecule as a template or an information source (e.g., synthesizing a cDNA using a magnetically immobilized mRNA; synthesizing a polypeptide using a magnetically immobilized mRNA; synthesizing a DNA using a magnetically immobilized DNA, and the like).

As noted above, a biomolecule may comprise two or more moieties belonging to different classes of biomolecules, e.g., polypeptides, polynucleotides, lipids, saccharides. A modification may be directed at only one moiety of a biomolecule. Thus, e.g., where the biomolecule is a peptide nucleic acid, a method of modifying a polypeptide applies to the peptide portion of the biomolecule. Accordingly, "modification of a polypeptide" includes modification of a biomolecule that is entirely a polypeptide, and modification of the polypeptide portion of a biomolecule that comprises, in addition to a polypeptide moiety, a non-polypeptide moiety.

In many embodiments, the modification is an enzymatic modification. In some of these embodiments, the enzyme is added in solution to the separation device. In other embodiments, the enzyme is immobilized in the separation device. In some of these embodiments, the enzyme is maintained in an inactive state before and/or after the modification reaction. Enzymes can be maintained in an inactive state by reducing the temperature to below a temperature at which the enzyme is active; by including an inhibitor of the enzyme; by using an apoenzyme that is inactive until activated by a cofactor; by deprivation of an ion that is required for enzymatic activity (e.g., $Ca^{2+}$, $Mg^{2+}$, etc.); by adjusting the pH to a pH at which the enzyme is inactive; and the like. After the enzymatic reaction, the enzyme can be inactivated by raising or lowering the temperature; adding an inhibitor of the enzyme; proteolytically digesting the enzyme; by deprivation of an ion that is required for enzymatic activity (e.g., $Ca^{2+}$, $Mg^{2+}$, etc.); by adjusting the pH to a pH at which the enzyme is inactive; and the like. An enzyme can be immobilized in the separation device by binding the enzyme (covalently or non-covalently, directly or through a linker) to a matrix material, e.g., a bead or other solid support.

Where the biomolecule or moiety of a biomolecule is a polypeptide, modifications to polypeptides include modifications by enzymatic reactions and modifications by non-enzymatic reactions. Modifications of polypeptides include, but are not limited to, binding to other polypeptides; deglycosylation; glycosylation; phosphorylation; dephosphorylation; nitrosylation; nucleotidylation; acylation; acetylation; ADP-ribosylation; methylation; ubiquitination; oxido shuffling (e.g., disulfide bridge formation in the presence of redox substances); labeling (e.g., with a detectable label); lipidation (e.g., myristilation); carboxylation; hydroxylation; proteolytic cleavage to remove portions of a polypeptide; proteolytic cleavage to cleave a protein into specific fragments; addition of tags, e.g. polyhistidine, epitope tags, and the like; binding to nucleic acid molecules; and labeling with detectable labels.

Detectable labels include direct labels or indirect labels. Direct labels include radioisotopes; enzymes whose products are detectable (e.g., luciferase, β-galactosidase, and the like); fluorescent labels (e.g., fluorescein isothiocyanate, rhodamine, phycoerythrin, a cyanine dye, Cascade Blue, Cy5, allophycocyanin, Cy5PE or other tandem conjugates of different fluorochromes, Texas Red, and the like); fluorescence emitting metals, e.g., $^{152}$Eu, or others of the lanthanide series, attached to the protein through metal chelating groups such as EDTA; chemiluminescent compounds, e.g., luminol, isoluminol, acridinium salts, and the like; bioluminescent compounds, e.g., luciferin, aequorin (green fluorescent protein), and the like; and metallic compounds. Indirect labels include labeled molecules that bind to the polypeptide, e.g., antibodies specific for the polypeptide, wherein the labeled binding molecule is labeled as described above; and members of specific binding pairs, e.g., biotin, (a member of the specific binding pair biotin-avidin), digoxigenin (a member of the specific binding pair digoxigenin-antibody to digoxigenin) and the like.

Where the biomolecule or moiety of a biomolecule is a polynucleotide, modifications to polynucleotides include modifications by enzymatic reactions and modifications by non-enzymatic reactions. Modifications of polynucleotides include, but are not limited to, synthesis of double-stranded nucleic acid molecules using a single-stranded nucleic acid molecule as template, e.g., by the action of a reverse transcriptase, a thermostable DNA polymerase (e.g., Taq DNA polymerase from *Thermus aquaticus,* Vent DNA polymerase from *Thermococcus litoralis,* Pfu DNA polymerase from *Pyrococcus furiosus;* or a non-thermostable DNA polymerase); addition of one or more nucleotides to the 5' and/or 3' end of a polynucleotide, e.g., by the action of polynucleotide kinase, terminal transferase, or Poly(A) polymerase; incorporation of polynucleotides, e.g., by Klenow fragment of a DNA polymerase (e.g., *E. coli* DNA polymerase 1) into a polynucleotide; ligation of a single-stranded or double-stranded linker or adaptor (e.g., a double-stranded linker with a single-stranded overhang) to a polynucleotide, e.g., for cloning purposes; restriction of a polynucleotide (e.g., by a restriction endonuclease); modification of one or both ends of a polynucleotide, including, but not limited to, phosphorylation; dephosphorylation; base removal (e.g., with mung bean nuclease, and the like); elongation of a polynucleotide, e.g., by a telomerase; introduction of one or more mutations into a polynucleotide; transcription and/or translation of a polynucleotide (e.g., using RNA polymerase or yeast extract); nicking or degradation of nucleic acids (e.g., using RNaseH after reverse transcriptase in cDNA synthesis); recombination of a polynucleotide, e.g., using creloxP system; methylation of nucleic acids; removal of a subgroup of components and/or reactants used for the modifying reaction during an enzymatic reaction, e.g., by action of a DNase and/or a protease); hybridization of a polynucleotide with one or more other nucleic acid molecules; binding of polypeptides to a polynucleotide (e.g., binding of a transcription factor(s), a translation factor(s), and the like); and detectably labeling a polynucleotide. Detectable labels for polynucleotides include direct labels and indirect labels, and include labels as described above for polypeptides.

Enzymatic modifications are conducted at a temperature at which the enzyme exhibits at least about 10%, at least about 20%, at least about 30%, at least about 40%, at least about 50%, at least about 60%, at least about 70%, at least about 80%, at least about 90%, or at least about 95% of its maximal activity. In some embodiments, enzymatic modifications are conducted at or near the temperature optimum for a given enzyme. Temperature optima for a wide variety of modifying enzymes are well known in the art. Temperature optima depend, in part, on the organism from which the enzyme is derived, and specific attributes of the particular enzyme. Thus, e.g., commercially available DNA ligase derived from T4 bacteriophage has a temperature optimum of about 16°C, while DNA ligase derived from *Thermus aquaticus* has a temperature optimum of about 45°C. The term "at or near the temperature optimum," as used herein, refers to a temperature that is within about 5%, within about 10%, or within about 15%, of the temperature optimum for a given enzyme, as long as the enzyme retains at least about 10%, at least about 20%, at least about 30%, at least about 40%, at least about 50%, at least about 60%, at least about 70%, at least about 80%, or at least about 90% of its maximal activity.

As used herein, the term "modification" includes hybridization of a biomolecule comprising a nucleotide sequence to an immobilized polynucleotide. Thus, in some embodiments, the methods comprise immobilizing a biomolecule comprising a polynucleotide; and contacting a second biomolecule comprising a polynucleotide with the immobilized polynucleotide under conditions that favor hybridization. At least a portion of the second biomolecule has substantial complementarity to the immobilized polynucleotide such that hybridization can occur. As one non-limiting example, the immobilized biomolecule may comprise an oligo d(T) tract of from about 6 to about 50, from about 8 to about 40, from about 10 to about 30, or from about 12 to about 20 nucleotides in length; and the second biomolecule may comprise a contiguous stretch of from about 6 to about 50, from about 8 to about 40, from about 10 to about 30, or from about 12 to about 20 adenosine residues.

As another non-limiting example, the immobilized polynucleotide may have from about 6 to about 50, from about 8 to about 40, from about 10 to about 30, or from about 12 to about 20 nucleotides that are substantially complementary with a corresponding stretch of contiguous nucleotides in a second biomolecule. An immobilized polynucleotide may be a full-length cDNA molecule, e.g., in subtractive hybridization applications. Accordingly, an immobilized polynucleotide may have from about 100 to about 1000, from about 1000 to about 2000, from about 2000 to about 3000, from about 3000 to about 5000, or from about 5000 to about 10,000, or more, nucleotides that are substantially complementary with a corresponding stretch of contiguous nucleotides in a second biomolecule.

As used herein, the term "modification" includes use of an immobilized polynucleotide (e.g., an immobilized biomolecule comprising a polynucleotide) as a template for synthesizing a polynucleotide or a polypeptide. Thus, in some embodiments, the methods comprise immobilizing a biomolecule comprising a polynucleotide, wherein the biomolecule is bound, directly or indirectly, to a magnetic particle on a magnetic separation column; and synthesizing a polynucleotide having a sequence that is substantially complementary to the immobilized polynucleotide.

Conditions for synthesizing a polypeptide using an immobilized biomolecule comprising an mRNA as a template are well known in the art; for synthesizing a cDNA using an immobilized biomolecule comprising an mRNA as a template; and for synthesizing a polynucleotide using an immobilized biomolecule comprising a DNA molecule as a template are well known in the art and need not be elaborated upon herein. Modification enzymes that may be contacted with the immobilized polynucleotide include a reverse transcriptase, e.g., where the immobilized polynucleotide comprises an mRNA molecule; a DNA polymerase, such as a thermostable DNA polymerase, e.g., where the immobilized polynucleotide comprises a DNA molecule. In some embodiments, a synthesis reaction may comprise multiple synthesis steps. Thus, e.g., where the immobilized biomolecule comprises a DNA molecule, a polymerase chain reaction (PCR) may be carried out.

PCR methods are well known in the art and are described in numerous publications, including, e.g., PCR2: A Practical Approach (1995) M.J. McPherson et al., eds. Oxford Univ. Press. A non-limiting example of PCR reaction conditions is the following: denaturation at from about 90°C to about 99°C, from about 92°C to about 96°C, or about 94°C for 30 seconds to 2 minutes; annealing at about 55°C for about 10 seconds to about 30 seconds; and extension at from about 60°C to about 70°C, or about 72°C for about 15 seconds to 1 minute. The denaturation, annealing, and extension steps may be repeated any number of times, where one denaturation, annealing, and extension series is a "cycle," any number of cycles can be performed, e.g., from about 2 to about 50, from about 4 to about 40, or from about 8 to about 25 cycles.

Capturing a newly synthesized or modified biomolecule

The methods of the invention result in generation of a modified biomolecule, or a newly synthesized biomolecule. In some of these embodiments, the newly synthesized or modified biomolecule is captured by a second binding moiety (a "capture moiety") in the separation device. The capture moiety is immobilized on the matrix, such that the captured biomolecule is also immobilized. In some of these embodiments, the captured biomolecule is further modified, or is purified without modification.

In some embodiments, the modification step results in a newly synthesized polypeptide, and the newly synthesized polypeptide is captured by a capture moiety. The capture moiety can be a member of a specific binding pair that binds specifically to the polypeptide. Suitable capture moieties include, but are not limited to, a ligand for the polypeptide; an antibody specific for the polypeptide; a polypeptide to which the newly synthesized polypeptide specifically binds; a nucleic acid to which the newly synthesized polypeptide specifically binds; and the like.

In some embodiments, the newly synthesized polypeptide includes a tag fused inframe to the carboxyl terminus, the amino terminus, or internally to the polypeptide, and the capture moiety is a molecule that binds to the tag.

In some embodiments, the tag is an immunological tag (an "epitope tag"). Immunological tags are known in the art, and are typically a sequence of between about 6 and about 50 amino acids that comprise an epitope that is recognized by an antibody specific for the epitope. Non-limiting examples of such tags are hemagglutinin (HA; e.g., CYPYDVPDYA; SEQ ID NO:1), FLAG (e.g., DYKDDDDK; SEQ ID NO:2), c-myc (e.g., CEQKLISEEDL; SEQ ID NO:3), and the like. In these embodiments, the capture moiety is an antibody specific for the epitope tag.

In other embodiments, the tag is a metal ion chelating tag, e.g., a polyhistidine tag (e.g., 2-20, 2-10, or 2-5 consecutive histidine residues; or a sequence of from about 10 to about 20 amino acids comprising at least about 30% histidine residues; and the like), and the capture moiety is a nickel or cobalt chelating ligand. Metal ion chelating tags and suitable ligands are described in the literature. See, e.g., U.S. Patent Nos. 5,594,115; 5,284,933; 5,047,513; and 5,310,663.

In some embodiments, a proteolytic cleavage site is disposed between the tag and the remainder of the newly synthesized polypeptide. In these embodiments, the newly synthesized is captured on the capture moiety, and, following capture, the tag is proteolytically cleaved from the remainder of the polypeptide. The remainder of the polypeptide can then be eluted. In some of these embodiments, the enzyme that carries out the proteolytic cleavage is immobilized on the column (as described herein), such that the enzyme that carries out the proteolytic cleavage does not contaminate the eluted polypeptide.

Proteolytic cleavage sites are known to those skilled in the art; a wide variety are known and have been described amply in the literature, including, e.g., Handbook of Proteolytic Enzymes (1998) AJ Barrett, ND Rawlings, and JF Woessner, eds., Academic Press. Proteolytic cleavage sites include, but are not limited to, an enterokinase cleavage site: $(Asp)_4Lys$ (SEQ ID NO:4); a factor Xa cleavage site: Ile-Glu-Gly-Arg (SEQ ID NO:5); a thrombin cleavage site, e.g., Leu-Val-Pro-Arg-Gly-Ser (SEQ ID NO:6); a renin cleavage site, e.g., His-Pro-Phe-His-Leu-Val-Ile-His (SEQ ID NO:7); a collagenase cleavage site, e.g., X-Gly-Pro (where X is any amino acid); a trypsin cleavage site, e.g., Arg-Lys; a viral protease cleavage site, such as a viral 2A or 3C protease cleavage site, including, but not limited to, a protease 2A cleavage site from a picornavirus (see, e.g., Sommergruber et al. (1994) Virol. 198:741-745), a Hepatitis A virus 3C cleavage site (see, e.g., Schultheiss et al. (1995) J. Virol. 69:1727-1733), human rhinovirus 2A protease cleavage site (see, e.g., Wang et al. (1997) Biochem: Biophys. Res. Comm. 235:562-566), a picornavirus 3 protease cleavage site (see, e.g., Walker et al. (1994) Biotechnol. 12:601-605; and a caspase protease cleavage site, e.g., DEVD (SEQ ID NO: 8) recognized and cleaved by activated caspase-3, where cleavage occurs after the second aspartic acid residue. In some embodiments, from 2 to about 12, or from about 4 to about 8, additional amino acids on the carboxyl and/or amino terminus of the protease cleavage site are included, which additional amino acids are found in a native substrate of the protease.

In other embodiments, the newly synthesized biomolecule is a nucleic acid, and the capture moiety is a nucleic acid that is complementary to a portion of the newly synthesized nucleic acid. The newly synthesized nucleic acid can be a cDNA molecule (e.g., where the magnetically immobilized biomolecule is an mRNA, and a cDNA molecule is generated by a reverse transcriptase) or a DNA molecule (e.g., where the magnetically immobilized biomolecule is a DNA, and the newly synthesized DNA molecule is generated by a DNA polymerase reaction, such as a polymerase chain reaction).

In certain embodiments, the magnetically immobilized biomolecule is a member of a mixed population of nucleic acids, and the newly synthesized biomolecules are therefore a heterogeneous population of nucleic acids. The capture moiety is a polynucleotide, e.g., an oligonucleotide, that hybridizes specifically or preferentially (e.g., under stringent hybridization conditions) to a subset of the heterogeneous population, e.g., to a subset comprising nucleic acids that include a sequence that is substantially complementary to the oligonucleotide capture moiety.

In other embodiments, the capture moiety binds to a modified biomolecule, but not to the same biomolecule that does not contain the modification. Such capture moieties include, but are not limited to, anti-phosphotyrosine antibodies (binding to phosphorylated tyrosine residues of a protein); avidin (binding to biotinylated biomolecule); ligands specific for a modification; antibody specific for a modification; and the like.

### Temperature

In some embodiments, the temperature of the separation device or a portion of the separation device is altered before and/or during and/or after modification of a biomolecule immobilized in the separation device. The temperature of the separation device is controlled to achieve a desired effect. The apparatus (or a portion thereof where the magnetically labeled biomolecule that is to be modified is immobilized) is maintained at a given temperature for a period of time sufficient to achieve the desired effect

It is well within the skill level of those skilled in the art to determine the period of time that is sufficient to achieve the desired effect. For example, for an enzymatic modification, the manufacturer's suggestions for suitable time period may be followed, or the suitable time period may be determined by measuring the amount of product produced by the enzymatic reaction in a given period of time. Typically, between about 30 seconds and 60 minutes will be sufficient for most enzymatic reactions. For binding reactions (e.g., protein-protein interactions, protein-nucleic acid interactions, nucleic acid-nucleic acid hybridizations) those skilled in the art can readily determine suitable time periods. For example, suitable time periods for nucleic acid-nucleic acid hybridizations range from about 1 minute to about 60 minutes.

The temperature of the device can be altered (adjusted) one or more times to achieve various effects.

The temperature of the separation device can be altered to affect modification of a biomolecule, including, but not limited to, to affect hybridization of two nucleic acid molecules, e.g., to effect hybridization, to effect dehybridization; to slow down or stop an enzymatic reaction, e.g., by changing the temperature to a temperature that is above or below the optimal temperature for the enzyme; to allow an enzymatic reaction to proceed; to provide optimal activity for a modifying enzyme; to alter viscosity of the fluid, to reduce fluid volume (e.g., by evaporation); to increase enzyme concentration or salt concentration of the buffer (by evaporation); to initiate a reaction (e.g., an enzyme is in an inactive state due to binding to an agent; to initiate the enzymatic reaction, the temperature is increased to inactivate the blocking agent); to change the conformation of one or more biomolecules (e.g., increasing temperature to remove hairpin structures in RNA molecules; to denature double-stranded nucleic acid molecules; to elute a molecule (e.g., increase the temperature to elute a synthesized polynucleotide); and to affect binding of one biomolecules to another molecule.

The temperature of the separation device can be altered to affect hybridization of two nucleic acid molecules. Affecting hybridization of a nucleic acid molecule to a nucleic acid molecule immobilized on a separation device can be used to select nucleic acid molecules that bind to an immobilized nucleic acid molecule under specific conditions of hybridization stringency. Low stringency conditions may be used to identify homologs of a given nucleic acid molecule, e.g., nucleic acid molecules that share less than about 75%, less than about 70%, or less than about 65%, nucleotide sequence identity with an immobilized polynucleotide. High stringency conditions may be used to identify nucleic acid molecules that share at least about 75%, at least about 80%, at least about 85%, at least about 90%, or at least about 95%, or more, nucleotide sequence identity with an immobilized polynucleotide.

Hybridization reactions can be performed under conditions of different "stringency". Conditions that increase stringency of a hybridization reaction of widely known and published in the art. See, for example, Sambrook et al. (1989); and U.S. Patent No. 5,707,829. Examples of relevant conditions include (in order of increasing stringency): incubation temperatures of 25°C, 37°C, 50°C and 68°C; buffer concentrations of 10 x SSC, 6 x SSC, 1 x SSC, 0.1 x SSC (where SSC is 0.15 M NaCl and 15 mM citrate buffer) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6 x SSC, 1 x SSC, 0.1 x SSC, or deionized water. Examples of stringent conditions are hybridization and washing at 50°C or higher and in 0.1 x SSC (9 mM NaCl/0.9 mM sodium citrate).

"$T_m$" is the temperature in degrees Celsius at which 50% of a polynucleotide duplex made of complementary strands hydrogen bonded in anti-parallel direction by Watson-Crick base pairing dissociates into single strands under conditions of the experiment. $T_m$ may be predicted according to a standard formula, such as:

$$T_m = 81.5 + 16.6 \log[X^+] + 0.41 \, (\%G/C) - 0.61 \, (\%F) - 600/L$$

where [$X^+$] is the cation concentration (usually sodium ion, $Na^+$) in mol/L; (%G/C) is the number of G and C residues as a percentage of total residues in the duplex; (%F) is the percent formamide in solution (wt/vol); and L is the number of nucleotides in each strand of the duplex.

## Washing

The methods of the invention for modifying a biomolecule optionally comprise one or more washing steps. After the biomolecule is applied to a separation device, and before modification of the immobilized biomolecule, one or more washing steps may be performed. Washing may serve to remove unbound components. After modification of the biomolecule, one or more washing steps may be performed. Such washing steps may serve various functions, including: removal of modifying components of the modification reaction; removal of unwanted by-products of the modification reaction; stopping a particular modification reaction; exchanging a buffer; desalting; removal of nucleic acid fragments; removal of enzymes; removal of cofactors; removal of proteins; removal of non-specifically bound molecules; removal of inhibitors that result from reactions carried out in the device (e.g., removal of pyrophosphate, a product of polymerase or reverse transcriptase reaction); changing the pH; and stabilization of an intermediate. In addition, where more than one modification step is performed, the separation device may be washed in between steps.

The composition and temperature of a washing solution may vary according to the desired result. The optimal composition and temperature of a washing solution can readily be determined by those skilled in the art, based on known properties of the immobilized biomolecule and/or a molecule that is bound to the immobilized biomolecule.

Wash solutions may comprise a buffer, and may further comprise additional components, as necessary, including, but not limited to, a chelating agent, e.g., EGTA, EDTA; a detergent, e.g., sodium dodecyl sulfate, Triton X-100; CHAPS, etc.; various ions, e.g., $Ca^{++}$, $Mg^{++}$, $K^+$, $Ni^+$, etc.; reducing agents (e.g., DTT, DTE, β-mercaptoethanol, and cysteine); salts; glycerol; tRNA; nuclease inhibitors; protease inhibitors; cofactors; polyamines; nucleotides; nucleotide analogs; glycogen; albumin; imidazole; denaturing agents (e.g., urea, guanidinium chloride, and the like); peptides (e.g., glutathione); etc.

### Eluting

The immobilized biomolecule or other component may be eluted from the separation device after a modification procedure(s). In some embodiments, the immobilized biomolecule is retained on the column, and only a product of a modification reaction is eluted. In other embodiments, both the immobilized biomolecule and a product of a modification reaction (where the product of the modification reaction is other than the immobilized biomolecule) are eluted. In still other embodiments, where the immobilized biomolecule is modified by a modification reaction, only the modified immobilized biomolecule is eluted. The biomolecule to be immobilized can contain, or can be modified to contain, a site for proteolytic cleavage, or a site for cleavage by a restriction endonuclease, such that, when desired, e.g., after one or more modification steps, the modified immobilized biomolecule can be contacted with an appropriate enzyme (e.g., a proteolytic enzyme that specifically acts on the proteolytic cleavage site; a restriction endonuclease that acts on the restriction endonuclease recognition site), and the modified immobilized biomolecule can be released from the column. The biomolecule can be eluted together with the magnetic particle or separately from the magnetic particle, e.g., the magnetic particle is retained on the column, while the biomolecule is released from the magnetic particle.

### UTILITY

The methods and apparatus of the invention are useful in a wide variety of applications. Such applications include, but are not limited to, generation of labeled cDNA probes for use in probing DNA arrays; serial analysis of gene expression (SAGE) applications; and the like.

One non-limiting example of an application in which the methods of the invention find utility include generation of populations of labeled cDNA for use as probes for DNA-based arrays, e.g., to identify cDNAs expressed in response to an external or internal signal. In such applications, a population of detectably labeled cDNA can be synthesized and purified on a single apparatus as described herein. The apparatus may have multiple columns, each of which is used to synthesize a population of cDNA from a cell or cell population exposed to a different external or internal signal that affects gene expression. The labeled cDNA probes are then used to hybridize with arrays of DNA molecules, and hybridization with a labeled probe is detected using known methods. DNA arrays and their uses are amply described in the literature.

External and internal signals that affect gene expression include, but are not limited to, infection of a cell by a microorganism, including, but not limited to, a bacterium (e.g., *Mycobacterium* spp., *Shigella, Chlamydia,* and the like), a protozoan *(e.g., Trypanosoma* spp., *Plasmodium* spp., *Toxoplasma* spp., and the like), a fungus, a yeast (e.g., *Candida* spp.), or a virus (including viruses that infect mammalian cells, such as human immunodeficiency virus, foot and mouth disease virus, Epstein-Barr virus, and the like; viruses that infect plant cells; etc.); change in pH of the medium in which a cell is maintained or a change in internal pH; excessive heat relative to the normal range for the cell or the multicellular organism; excessive cold relative to the normal range for the cell or the multicellular organism; an effector molecule such as a hormone, a cytokine, a chemokine, a neurotransmitter; an ingested or applied drug; a ligand for a cell-surface receptor; a ligand for a receptor that exists internally in a cell, e.g., a nuclear receptor; hypoxia; light; dark; mitogens, including, but not limited to, lipopolysaccharide (LPS), pokeweed mitogen; antigens; sleep pattern; electrical charge; ion concentration of the medium in which a cell is maintained or an internal ion concentration, exemplary ions including sodium ions, potassium ions, chloride ions, calcium ions, and the like; presence or absence of a nutrient; metal ions; disregulation of cell cycle; a transcription factor; a tumor suppressor; cell-cell contact; and the like.

SAGE applications have been described in the art. See, e.g., U.S. Patent Nos. 5,695,937; 5,866,330; 6,221,600; 6,261,782; and 6,297,017. As one non-limiting example, a population of double-stranded cDNAs are synthesized, using, e.g., a biotinylated oligo dT primer; the biotinylated ds cDNAs are applied to a separation device of the invention that includes avidin-bound magnetic particles such that the ds cDNA molecules are immobilized; the immobilized cDNA molecules are cleaved with a restriction endonuclease; the cleaved cDNA molecules are ligated with double-stranded adapter molecules, which may include an overhanging end that anneals with an overhanging end of the ds cDNA molecule and regenerates the restriction site, and which may also include an overhanging end that serves as a primer; and the population of ligated molecules are released from the device. The population of molecules can then be used in any SAGE application.

## EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

Example 1: Probe generation for microarray hybridization

DNA microarrays are used to analyze and compare the expression level of a gene in different cell types or tissues, or in response to particular conditions. For these applications, i.e., expression profiling, mRNA is generally reverse transcribed into cDNA and thereby modified by an introduction of labeled nucleotides. Two populations of cDNA molecules, each labeled with a different detectable label, are hybridized to DNA microarrays and compared with respect to their signal intensities, which reflects the expression pattern of corresponding genes.

The probe generation for microarray hybridization is a complex process which consists of a variety of steps, including mRNA isolation, cDNA synthesis and cDNA labeling, RNaseH digestion and purification of the labeled cDNA. Using magnetic particles, a separation column and a temperature regulatable magnet, all these steps are carried out in one matrix.

For mRNA isolation, cells were processed according to the mRNA isolation protocol from Miltenyi Biotec which uses magnetic particles and columns in a magnetic field for separation purposes. In brief, mRNA was released by cell lysis using a lysis buffer system and cell debris was removed with a filter unit, resulting in a homogeneous cell lysate. The poly(A) tail of mRNA molecules was hybridized to oligo(dT) sequences of small magnetic microbeads and applied to a MACS separation column type μ. For purification of magnetically immobilized mRNA molecules, a series of washing steps was carried out to remove contaminating molecules such as DNA, proteins, and ribosomal RNA. Washing with lysis buffer was followed by several washing steps with wash buffer for more stringent purification. Afterwards, mRNA remained immobilized on the column.

cDNA was synthesized using Superscript II (Life technologies), Expand Reverse Transcriptase (Roche), Stratascript (Stratagene) or Omniscript (Qiagen). In addition to reverse transcriptase, the reaction mixture contained a set of unlabeled desoxynucleotides - dATP, dCTP, dGTP, dTTP - (Life Technologies, Roche, Qiagen) in a final concentration of up to 1 mM; Cy3 or Cy5 labeled dCTP (Amersham Pharmacia Biotech) in a final concentration of 0.1 mM; dithiothreitol in a final concentration of 10 mM; and RNase inhibitor (Roche, Life Technologies) in an appropriate buffer system. Before cDNA synthesis, mRNA was equilibrated with 2x100 μl buffer for reverse transcription. After equilibration 20-30 μl of the above-mentioned reaction mixture was applied to the μ-column and reverse transcription was started turning the heating facility of the magnet to the temperature optimum of the enzyme, which lies at 37°C or 42°C, depending on the enzyme used and following manufacturer's specifications for temperature optima. After an incubation time between 45 and 60 minutes. the synthesis was stopped and the cDNA was purified at the same time by applying 2x100 μl reverse transcriptase buffer on the μ column.

Commonly used reverse transcriptases lack RNase H activity or have only a reduced RNase activity. Therefore, most cDNA molecules are bound to their mRNA template. To remove the mRNA, 20-30 μl of a solution containing RNase H (Roche, Life Technologies) in an appropriate buffer system was applied to the μ-column and reaction was started-by turning the heating facility of the magnet to 37°C. After 25 minutes digestion was completed and residual mRNA fragments were removed by two washing steps with 100 μl phosphate buffered saline.

After these final washing steps, the fluorescently labeled single stranded pure cDNA was magnetically immobilized on the μ-column. To elute the cDNA, 20-30 μl of a release reagent (Miltenyi Biotec) which separates the cDNA from the magnetic particles was applied to the column and after a 10-minute incubation at room temperature, cDNA was eluted in a Tris-EDTA based buffer system. The pure cDNA was precipitated, dissolved in hybridization buffer and hybridized against DNA microarrays according to the manufacturer's instructions (GeneScan Europe).

Example 2: Dnase I activity

mRNA molecules are used for a variety of applications where the gene expression of cells or tissues is analyzed. After isolation of mRNA by hybridization of oligo(dT) sequences to the poly(A) tail of transcripts the mRNA is very clean but not free of minute amounts of genomic DNA which results mainly from nonspecific binding of oligo(dT) sequences to intramolecular Adenosin stretches. In most downstream applications, this genomic DNA does not interfere with the results, but for some applications, such as generation of cDNA expression libraries, the mRNA has to be free of genomic contaminants. Therefore, in such applications mRNA isolation is followed by DNase I digestion. To avoid intensive purification steps after the enzymatic reaction the DNase I digestion was carried out on a column which allows an easy purification of the mRNA after digestion.

mRNA was isolated using oligo(dT) microbeads (Miltenyi Biotec) according to the manufacturer's instructions. After extensive washing, mRNA was not eluted from the μ-column but instead was equilibrated once with an appropriate reaction buffer for DNase I enzyme. Equilibration was followed by the reaction itself by applying 20-30 μl reaction mixture to the column. The reaction mixture contained DNase I in an appropriate buffer system (Roche). After an incubation for 10 minutes at room temperature or 30°C, respectively, the mRNA was washed again with 2x200 μl lysis buffer and 2x200 μl washing buffer used for mRNA purification (Miltenyi Biotec). For elution 120 μl of elution buffer was applied to the column. Drops 2-4 contain the pure mRNA which is free of contaminating genomic DNA.

Example 3: Ligation of linkers and restriction of DNA in the course of applying SAGE technology

There are a number of complex techniques which involve the modification of biotinylated nucleic acids immobilized via streptavidin binding units. An application in which a series of reactions is carried out during an immobilization is SAGE technology in which thousands of transcripts are analyzed in detail with respect to their expression status (Velculescu et al. (1995) Science 270 :484-7).

Starting with mRNA, double-stranded cDNA was synthesized using biotinylated oligo(dT) primer. After synthesis, cDNA was cleaved with an appropriate restriction enzyme. To create fragments of an optimal size, usually restriction endonucleases with a 4-basepair recognition site were used.

Two MACS columns type μ (Miltenyi Biotec) were placed in the magnetic field of a MACS separator. The columns were prepared by rinsing each with 100 μl of equilibration buffer for nucleic acids (Miltenyi Biotec) and two times with 100 μl binding buffer (500, mM NaCl, 1 mM EDTA, 10 mM Tris-HCl, pH 8.0). After preparation of the columns MAGmol streptavidin microbeads were added to the solution containing the biotinylated cDNA. To create optimal binding conditions, binding was carried out in the above mentioned binding buffer in a final volume between 100 and 500 μl. The capturing of biotinylated restriction fragments was generally completed after a few seconds. One half of the binding solution was applied to each μ-column and washed three to four times with binding buffer.

For ligation of linkers, 20-30 μl of the ligation mixture was applied onto the μ-column and incubated for at least 3 hours at 16°C. Therefore, using the cooling facility of the magnet, the temperature of the column was adjusted. To remove the non-ligated linkers and all other components of the ligation reaction, the column was washed three to four times with 100 μl binding buffer.

For the next restriction step, which separates linker-ligated cDNA fragments from magnetic particles and so also from the column, a mixture containing the restriction endonuclease was applied onto the μ-column in a volume of 20-30 μl. The temperature of the column was adjusted to the optimal reaction temperature of the enzyme which lies generally at 65°C or 37°C (depending on the enzyme) by turning the heating facility of the magnet on.

For elution of the restricted DNA fragment, at least 100 μl of buffer was applied onto the column. Additional steps were performed according to standard protocols.

Example 4: Biotinylation and isolation of DNA

The binding of biotin to streptavidin is one of the strongest biological non-covalent interactions. Therefore, to remove biotinylated DNA from μMACS Streptavidin MicroBeads, an enzymatic reaction with a restriction endonuclease on the column placed in the magnetic field of MACS Separator can be carried out. The immobilized biotinylated DNA is enzymatically cleaved with a restriction endonuclease that cleaves at a restriction site that is close to the biotin group. The necessary temperature for the enzymatic reaction can be obtained with a heatable μMACS separation device or by incubation of the whole separation unit in an appropriate incubator. The biotinylated fragment is retained by μMACS Streptavidin MicroBeads, while the unbiotinylated DNA formed by action of the restriction endonuclease can be washed out and collected. Elution of the biotinylated fragment is also possible.

*Generation of a biotinylated DNA*

A known DNA sequence is amplified and biotinylated at the same time using one 5' biotinylated primer in a PCR reaction. The DNA is biotinylated is at the end of the fragment which is to be retained by the μMACS Streptavidin MicroBeads (Miltenyi Biotec, Inc.). Alternatively, nucleic acids such as DNA plasmids are biotinylated using a commercially available biotinylation kits (e.g. with photobiotin).

*Binding of biotinylated DNA to μMACS Streptavidin MicroBeads*

The binding reaction is performed in the same reaction buffer as is used for the restriction endonuclease. A solution containing (1) DNA; (2) binding solution (5 μl of 10 x restriction enzyme buffer); and (3) μMACS Streptavidin MicroBeads; and (4) deionized water (dH$_2$O) to a final volume of 50 μl. The solution is mixed briefly and kept at room temperature for 2 minutes. 100 μl of the μMACS Streptavidin Microbeads generally bind up to about 100 pmol biotinylated oligonucleotides. The capturing of the DNA by μMACS MicroBeads is generally completed after a few seconds. If the temperature during capturing is lower than room temperature, the capturing time is extended up to 15 minutes. To get best results, the dilution of the μMACS Streptavidin Microbeads should be no more than 1:10.

*Preparation of the enzyme solution*

About 5-10 Units of the restriction enzyme per μg DNA (depending on the enzyme) are used. The enzyme is diluted in a suitable buffer (e.g., the buffer supplied by the manufacturer with the enzyme, or the buffer recommended by the manufacturer of the enzyme). The amount of enzyme for a restriction reaction on a column is the same as for a conventional restriction reaction in a tube. Typically, the enzyme solution contains: (1) 2 μl of 10 x restriction enzyme buffer; (2) x Units of the restriction enzyme; and (3) dH$_2$O to a total of 20 μl. Incubate the solution for 1 hour at room temperature.

*Preparation of the column*

A MACS μ column is placed in the magnetic field of a (heatable) μMACS separation device. The column is prepared by rinsing with 100 μl of equilibration buffer for protein applications and with 2 x 100 μl of 1x reaction buffer of the restriction enzyme.

*Restriction digestion*

The binding solution is applied to the top of the column matrix and is allowed to pass through. The magnetically labeled DNA is retained in the column. Afterwards, the enzyme solution is applied to the top of the column. The heatable magnet is adjusted to 37°C or other temperature appropriate to the restriction enzyme (alternatively, the column with the magnet is put in an incubator at the desired temperature). The device is kept at the appropriate temperature for 1 hour.

*Elution*

The heatable magnet is turned off (or the column with the magnet is taken out of the incubator). To elute the unbiotinylated DNA fragment, the column is washed with 200 μl of a suitable buffer (e.g. TE pH 8.0), and fractions collected. To elute the biotinylated DNA fragment, the column is taken out of the magnet, and 200 μl of an appropriate buffer (e.g. TE pH 8.0) is applied.

*Analysis*

Before analysis, DNA is precipitated by ethanol precipitation to reduce the volume. If necessary, DNA is released from the microbeads by incubating with 0.1 % SDS at 95°C for 5 minutes, immediately followed by a centrifugation for 1 minute at 15,000 x g; the supernatant (containing DNA) is then transferred to a fresh tube; and the procedure is repeated twice.

**Claims**

**1.** A method for modifying a biomolecule, comprising:

a) immobilizing a biomolecule bound to a magnetic particle on a magnetic separation apparatus comprising at least one separation chamber by applying a magnetic field to a magnetizable matrix (210) in the separation chamber; wherein the separation apparatus further comprises a separation unit (302) provided with a controllable heat and/or cooling source (332) for controlling the temperature within the at least one separation chamber *in situ,* and

b) modifying the immobilized biomolecule, wherein one or more modification steps are conducted at a temperature that is suitable for modification.

2. The method of claim 1, further comprising eluting the modified biomolecule from the separation chamber.

3. The method of claim 1 or 2, wherein the modification is an enzymatic modification with at least a first enzyme, and the apparatus is maintained for a first period of time at a first temperature at which the first enzyme exhibits at least 10% of its maximal activity.

4. The method of claim 3, further comprising:

c) modifying the immobilized biomolecule with a second enzyme, wherein the apparatus is maintained for a second period of time at a second temperature at which the second enzyme exhibits at least 10% of its maximal activity.

5. The method of anyone of the preceding claims, wherein the biomolecule comprises a polypeptide, and the modification is selected from the group consisting of phosphorylation, dephosphorylation, nitrosylation, acetylation, deglycosylation, glycosylation, acylation, methylation, ADP riboxlation, ubiquitination, lipidation, carboxylation, hydroxylation, and nucleotidylation.

6. The method of anyone of the preceding claims, wherein the biomolecule comprises a polypeptide, and the modification is labeling with a detectable label.

7. The method of claim 1 or 2, wherein the immobilized biomolecule comprises a polynucleotide, and the modification comprises hybridization to a second biomolecule comprising a polynucleotide comprising a nucleotide sequence that is substantially complementary to at least a portion of the immobilized polynucleotide.

8. The method of claim 7, wherein the immobilized biomolecule is a polynucleotide, and the modification comprises synthesizing a polynucleotide comprising a nucleotide sequence that is complementary to a nucleotide sequence in the immobilized polynucleotide.

9. The method of claim 1 or 2, wherein the immobilized biomolecule comprises a polynucleotide, and the modification is an enzymatic modification selected from the group consisting of synthesis of a polynucleotide complementary to the immobilized polynucleotide, addition of a nucleotide to the 5' end of the immobilized polynucleotide, addition of a nucleotide to the 3' end of the immobilized polynucleotide, ligation of a single-stranded polynucleotide to the immobilized polynucleotide, ligation of a double-stranded polynucleotide to the immobilized polynucleotide, cleavage of the immobilized polynucleotide at a restriction endonuclease recognition site, removal of a nucleotide from the immobilized polynucleotide, synthesis of a polypeptide using the immobilized polynucleotide as a template, and methylation of a base of a nucleotide of the immobilized polynucleotide.

10. The method of claim 1 or 2, wherein the immobilized biomolecule comprises a polynucleotide, and the modification is a non-enzymatic modification.

11. The method of claim 1 or 2, wherein the immobilized biomolecule comprises a polynucleotide, and the modification comprises binding a polypeptide to the immobilized polynucleotide.

12. The method of claim 1 or 2, wherein the immobilized biomolecule comprises a first polypeptide, and the modification comprises binding a second polypeptide to the immobilized polypeptide.

13. The method of claim 1 or 2, wherein the immobilized biomolecule comprises a double-stranded polynucleotide, and the modification comprises contacting the immobilized polynucleotide with a double-stranded polynucleotide of from about 6 to about 20 nucleotides in length, in the presence of a DNA ligase, at a temperature of about 16°C.

**14.** The method of claim 1, wherein the immobilized biomolecule comprises a polynucleotide and wherein the magnetic particle contains bound thereto an oligonucleotide that is complementary to a portion of the immobilized biomolecule and that serves as a primer for synthesis of a nucleic acid;

wherein in step (b) modifying comprises

i) contacting the immobilized polynucleotide with an enzyme that can synthesize a nucleic acid molecule, in the presence of deoxynucleotides, wherein the apparatus is maintained for a period of time at a temperature at which the enzyme exhibits at least 10% of its maximal activity; and

ii) synthesizing a nucleic acid molecule, using the immobilized polynucleotide as a template.

**15.** The method of claim 14, wherein at least one deoxynucleotide comprises a detectable label, and wherein the synthesized nucleic acid molecule comprises the at least one detectably labeled deoxynucleotide.

**16.** The method of claim 14 or 15, wherein the immobilized polynucleotide is an mRNA molecule, wherein the enzyme is a reverse transcriptase, wherein step (ii) is conducted at a temperature of from about 32°C to about 42°C, and wherein the synthesized nucleic acid molecule is a cDNA molecule.

**17.** The method of claim 16, further comprising:

c) contacting the cDNA molecule with RNaseR at a temperature of about 37°C; and

d) eluting the cDNA molecule.

**18.** The method of claim 1, wherein the immobilized biomolecule comprises a polynucleotide;

wherein in step (b) modifying comprises

i) contacting the immobilized polynucleotide with a first oligonucleotide primer and an enzyme that can synthesize a nucleic acid molecule, in the presence of deoxynucleotides, under conditions suitable for synthesis of a nucleic acid; and

ii) synthesizing a nucleic acid molecule, using the immobilized polynucleotide as a template.

**19.** The method of claim 18 wherein during step (b) the apparatus is maintained for a period of time at a temperature at which the enzyme exhibits at least 10% of its maximal activity.

**20.** The method of claim 18 or 19, wherein step (i) is conducted at a temperature of about 55°C, and/or wherein step (ii) is conducted at a temperature of from about 60°C to about 72°C.

**21.** The method of claim 18, 19 or 20, further comprising:

c) heating the separation chamber to a temperature to denature the synthesized nucleic acid;

d) contacting the synthesized nucleic acid molecule with a second oligonucleotide primer that hybridizes to a region in the synthesized nucleic acid molecule;

e) cooling the separation chamber to a temperature sufficient to allow hybridization of the second primer to the synthesized nucleic acid molecule such that hybridisation occurs; and

f) bringing the separation chamber to a temperature suitable for synthesis of a second nucleic acid from said second primer to occur.

**22.** The method of claim 21 wherein the heating of step (c) is from about 90°C to about 96°C; and/or the cooling (e) is to about 55°C; and/or the synthesis (f) is from about 60°C to about 72°C.

**23.** The method of claim 21 or 22, further comprising repeating cycles of denaturing, cooling and synthesising steps from 2 to about 30 times.

**24.** The method of claim 1, wherein the immobilized biomolecule comprises a polynucleotide comprising a poly(A) tract and the magnetic particle is bound to an oligo-dT molecule of from about 6 nucleotides to about 30 nucleotides;

wherein in step (b) modifying comprises

i) contacting the immobilized polynucleotide with an enzyme that can synthesize a nucleic acid molecule, in the presence of deoxynucleotides, wherein the apparatus is maintained for a period of time at a temperature at which the enzyme exhibits at least 10% of its maximal activity; and
ii) synthesizing a nucleic acid molecule, using the immobilized polynucleotide as a template.

25. The method of claim 24, wherein the immobilized polynucleotide is an mRNA molecule, and the synthesized nucleic acid molecule is a cDNA molecule.

26. The method of claim 25, further comprising contacting the immobilized mRNA and the synthesized cDNA molecule with RNAseH.

27. The method of claim 24, 25 or 26, wherein at least one of the deoxynucleotides comprises a detectable label, wherein the detectably labeled deoxynucleotide is incorporated into the synthesized nucleic acid molecule.

28. An apparatus for immobilizing and modifying biomolecules, comprising:

a separation unit (302), the unit (302) comprising:

a magnetic yoke (310) having at least one notch (312) formed therein;
a pair of magnets (314) placed within each of said at least one notch to form a gap (316) therebetween, said gap being adapted to receive a separation chamber therein; and
a controllable heat source and/or cooling source (332) thermally coupled to each said pair of magnets (314).

29. The apparatus of claim 28, further comprising at least one separation chamber having a wettable, flow through heat conducting matrix contained in each said separation chamber.

30. The apparatus of claim 29, wherein said wettable, flow through heat conducting matrix (210) is internally magnetizable.

31. The apparatus of any one of claims 28-30, further comprising an insulation layer (317) separating said magnets and said controllable heat source.

32. The apparatus of any one of claims 28-31, wherein each said controllable heat source also functions as a controllable cooling source.

33. The apparatus of any one of claims 28-31, further comprising a heat conducting element (338) thermally connecting each said controllable heating source with said respective pair of magnets.

34. The apparatus of claim 33, wherein each said heat conducting element (338) is configured to contact a separation chamber for conducting heat thereto.

35. The apparatus of any of claims 28 to 34, wherein at least one of said controllable heat sources comprises a heating film (332).

36. The apparatus of any of claims 28 to 35, wherein at least one of said controllable heat sources comprises a Peltier type heating source.

37. The apparatus of any of claims 28 to 36, wherein at least one of said controllable heat sources comprises a power resistance type heating source (432).

38. The apparatus of any of claims 28 to 37, wherein said controllable heat source comprises a pneumatic heating system.

39. The apparatus of any of claims 28 to 38, wherein said controllable heat source comprises a hydraulic heating system.

40. The apparatus of any of claims 28 to 39, wherein at least one of said controllable heat sources comprises a liquid driven element adapted to transfer heat from a liquid circulated therethrough.

41. The apparatus of any of claims 28 to 40, wherein at least one of said controllable heat sources comprises a radiant heating element.

**42.** The apparatus of claim 41, wherein each said radiant heating element comprises an infrared light emitting diode.

**43.** The apparatus of any of claims 28 to 42, wherein at least one of said controllable heat sources comprises an inductive heating element.

**44.** The apparatus of claim 43, wherein each said inductive heating element comprises a spool (1032) of wound wire.

**45.** The apparatus of any of claims 28 to 44, further comprising a controller (734) coupling each said controllable heat and/or cooling source (332) with a power source (1030), wherein said controller functions to control an amount of power delivered to each said controllable heat source to control a temperature thereof.

**46.** The apparatus of any of claims 28 to 45, further comprising a feedback sensor (348) associated with each said controllable heat source to provide feedback to said controller regarding a temperature of said respective controllable heat and/or cooling source.

**47.** The apparatus of claim 46, wherein each said feedback sensor comprises a thermocouple.

**Patentansprüche**

**1.** Verfahren zur Modifikation eines Biomoleküls, das Folgendes umfasst:

a) das Immobilisieren eines Biomoleküls, das an ein magnetisches Teilchen gebunden ist, an einer magnetischen Trennvorrichtung, die zumindest eine Trennkammer umfasst, durch das Anlegen eines Magnetfelds an eine magnetisierbare Matrix (210) in der Trennkammer, worin die Trennvorrichtung weiters eine Trenneinheit (302) umfasst, die mit einer steuerbaren Wärme- und/oder Kühlquelle (332) bereitgestellt ist, um die Temperatur in der zumindest einen Trennkammer in situ zu steuern, und
b) das Modifizieren des immobilisierten Biomoleküls, worin ein oder mehrere Modifikationsschritte bei einer für die Modifikation geeigneten Temperatur durchgeführt werden.

**2.** Verfahren nach Anspruch 1, das weiters das Eluieren des modifizierten Biomoleküls aus der Trennkammer umfasst.

**3.** Verfahren nach Anspruch 1 oder 2, worin die Modifikation eine enzymatische Modifikation mit zumindest einem ersten Enzym ist und die Vorrichtung über einen ersten Zeitraum hinweg auf einer ersten Temperatur gehalten wird, bei der das erste Enzym zumindest 10 % seiner Maximalaktivität aufweist.

**4.** Verfahren nach Anspruch 3, das weiters Folgendes umfasst:

c) das Modifizieren des immobilisierten Biomoleküls mit einem zweiten Enzym, worin die Vorrichtung über einen zweiten Zeitraum hinweg auf einer zweiten Temperatur gehalten wird, bei der das zweite Enzym zumindest 10 % seiner Maximalaktivität aufweist.

**5.** Verfahren nach einem der vorangegangenen Ansprüche, worin das Biomolekül ein Polypeptid umfasst und die Modifikation aus der aus folgenden bestehenden Gruppe ausgewählt ist: Phosphorylierung, Dephosphorylierung, Nitrosylierung, Acetylierung, Deglykosylierung, Glykosylierung, Acylierung, Methylierung, ADP-Ribosylierung, Ubiquitinierung, Lipidierung, Carboxylierung, Hydroxylierung und Nucleotidylierung.

**6.** Verfahren nach einem der vorangegangenen Ansprüche, worin das Biomolekül ein Polypeptid umfasst und die Modifikation im Markieren mit einer detektierbaren Markierung besteht.

**7.** Verfahren nach Anspruch 1 oder 2, worin das immobilisierte Biomolekül ein Polynucleotid umfasst und die Modifikation die Hybridisierung an ein zweites Biomolekül umfasst, das ein Polynucleotid umfasst, das eine Nucleotidsequenz umfasst, die zu zumindest einem Abschnitt des immobilisierten Polynucleotids im Wesentlichen komplementär ist.

**8.** Verfahren nach Anspruch 7, worin das immobilisierte Biomolekül ein Polynucleotid ist und die Modifikation das Synthetisieren eines Polynucleotids umfasst, das eine Nucleotidsequenz umfasst, die zu einer Nucleotidsequenz in dem immobilisierten Polynucleotid komplementär ist.

**9.** Verfahren nach Anspruch 1 oder 2, worin das immobilisierte Biomolekül ein Polynucleotid umfasst und die Modifikation eine enzymatische Modifikation ist, die aus der aus folgenden bestehenden Gruppe ausgewählt ist: die Synthese eines zu dem immobilisierten Polynucleotid komplementären Polynucleotids, das Hinzufügen eines Nucleotids an das 5'-Ende des immobilisierten Polynucleotids, das Hinzufügen eines Nucleotids an das 3'-Ende des immobilisierten Polynucleotids, das Ligieren eines einzelsträngigen Polynucleotids an das immobilisierte Polynucleotid, das Ligieren eines doppelsträngigen Polynucleotids an das immobilisierte Polynucleotid, das Spalten des immobilisierten Polynucleotids an einer Restriktionsendonucleaseerkennungsstelle, das Entfernen eines Nucleotids von dem immobilisierten Polynucleotid, die Synthese eines Polypeptids unter Verwendung des immobilisierten Polynucleotids als Matrize und die Methylierung einer Base eines Nucleotids des immobilisierten Polynucleotids.

**10.** Verfahren nach Anspruch 1 oder 2, worin das immobilisierte Biomolekül ein Polynucleotid umfasst und die Modifikation eine nicht-enzymatische Modifikation ist.

**11.** Verfahren nach Anspruch 1 oder 2, worin das immobilisierte Biomolekül ein Polynucleotid umfasst und die Modifikation das Binden eines Polypeptids an das immobilisierte Polynucleotid umfasst.

**12.** Verfahren nach Anspruch 1 oder 2, worin das immobilisierte Biomolekül ein erstes Polypeptid umfasst und die Modifikation das Binden eines zweiten Polypeptids an das immobilisierte Polypeptid umfasst.

**13.** Verfahren nach Anspruch 1 oder 2, worin das immobilisierte Biomolekül ein doppelsträngiges Polynucleotid umfasst und die Modifikation das Kontaktieren des immobilisierten Polynucleotids mit einem doppelsträngigen Polynucleotid mit einer Länge von etwa 6 bis etwa 20 Nucleotiden in Gegenwart einer DNA-Ligase bei einer Temperatur von etwa 16 °C umfasst.

**14.** Verfahren nach Anspruch 1, worin das immobilisierte Biomolekül ein Polynucleotid umfasst und worin das magnetische Teilchen ein daran gebundenes Oligonucleotid enthält, das zu einem Abschnitt des immobilisierten Biomoleküls komplementär ist und als Primer für die Synthese einer Nucleinsäure dient, worin das Modifizieren in Schritt (b) Folgendes umfasst:

   i) das Kontaktieren des immobilisierten Polynucleotids mit einem Enzym, das ein Nucleinsäuremolekül synthetisieren kann, in Gegenwart von Desoxynucleotiden, worin die Vorrichtung über einen Zeitraum hinweg auf einer Temperatur gehalten wird, bei der das Enzym zumindest 10 % seiner Maximalaktivität aufweist; und
   ii) das Synthetisieren eines Nucleinsäuremoleküls unter Verwendung des immobilisierten Polynucleotids als Matrize.

**15.** Verfahren nach Anspruch 14, worin zumindest ein Desoxynucleotid eine detektierbare Markierung umfasst und worin das synthetisierte Nucleinsäuremolekül das zumindest eine detektierbar markierte Desoxynucleotid umfasst.

**16.** Verfahren nach Anspruch 14 oder 15, worin das immobilisierte Polynucleotid ein mRNA-Molekül ist, worin das Enzym eine Umkehrtranskriptase ist, worin Schritt (ii) bei einer Temperatur von etwa 32 °C bis etwa 42 °C durchgeführt wird und worin das synthetisierte Nucleinsäuremolekül ein cDNA-Molekül ist.

**17.** Verfahren nach Anspruch 16, das weiters Folgendes umfasst:

   c) das Kontaktieren des cDNA-Moleküls mit RnaseR bei einer Temperatur von etwa 37 °C; und
   d) das Eluieren des cDNA-Moleküls.

**18.** Verfahren nach Anspruch 1, worin das immobilisierte Biomolekül ein Polynucleotid umfasst;
worin das Modifizieren in Schritt (b) Folgendes umfasst:

   i) das Kontaktieren des immobilisierten Polynucleotids mit einem ersten Oligonucleotid-Primer und einem Enzym, das ein Nucleinsäuremolekül synthetisieren kann, in Gegenwart von Desoxynucleotiden unter Bedingungen, die für die Synthese einer Nucleinsäure geeignet sind; und
   ii) das Synthetisieren eines Nucleinsäuremoleküls unter Verwendung des immobilisierten Polynucleotids als Matrize.

**19.** Verfahren nach Anspruch 18, worin die Vorrichtung während Schritt (b) über einen Zeitraum hinweg auf einer Temperatur gehalten wird, bei dem das Enzym zumindest 10 % seiner Maximalaktivität aufweist.

**20.** Verfahren nach Anspruch 18 oder 19, worin Schritt (i) bei einer Temperatur von etwa 55 °C durchgeführt wird und/ oder worin Schritt (ii) bei einer Temperatur von etwa 60 °C bis etwa 72 °C durchgeführt wird.

**21.** Verfahren nach Anspruch 18, 19 oder 20, das weiters Folgendes umfasst:

c) das Erhitzen der Trennkammer auf eine Temperatur zur Denaturierung der synthetisierten Nucleinsäure;
d) das Kontaktieren des synthetisierten Nucleinsäuremoleküls mit einem zweiten Oligonucleotid-Primer, der an eine Region in dem synthetisierten Nucleinsäuremolekül hybridisiert;
e) das Abkühlen der Trennkammer auf eine Temperatur, die ausreicht, um die Hybridisierung des zweiten Primers an das synthetisierte Nucleinsäuremolekül zu ermöglichen, damit es zu einer Hybridisierung kommt; und
f) das Herstellen einer Temperatur in der Trennkammer, die für das Eintreten der Synthese einer zweiten Nucleinsäure aus dem zweiten Primer geeignet ist.

**22.** Verfahren nach Anspruch 21, worin das Erhitzen in Schritt (c) auf eine Temperatur von etwa 90 °C bis etwa 96 °C erfolgt und/oder das Abkühlen (e) auf eine Temperatur von etwa 55 °C erfolgt und/oder die Synthese (f) bei einer Temperatur von etwa 60 °C bis etwa 72 °C erfolgt.

**23.** Verfahren nach Anspruch 21 oder 22, das ferner das 2- bis 30-malige Wiederholen von Zyklen von Denaturierungs-, Abkühlungs- und Synthetisierungsschritten umfasst.

**24.** Verfahren nach Anspruch 1, worin das immobilisierte Biomolekül ein Polynucleotid umfasst, das einen Poly(A)-Strang umfasst, und das magnetische Teilchen an ein Oligo-dT-Molekül mit etwa 6 bis etwa 30 Nucleotiden gebunden ist; worin das Modifizieren in Schritt (b) Folgendes umfasst:

i) das Kontaktieren des immobilisierten Polynucleotids mit einem Enzym, das ein Nucleinsäuremolekül synthetisieren kann, in Gegenwart von Desoxynucleotiden, worin die Vorrichtung über einen Zeitraum hinweg auf einer Temperatur gehalten wird, bei der das Enzym zumindest 10 % seiner Maximalaktivität aufweist; und
ii) das Synthetisieren eines Nucleinsäuremoleküls unter Verwendung des immobilisierten Polynucleotids als Matrize.

**25.** Verfahren nach Anspruch 24, worin das immobilisierte Polynucleotid ein mRNA-Molekül ist und das synthetisierte Nucleinsäuremolekül ein cDNA-Molekül ist.

**26.** Verfahren nach Anspruch 25, das ferner das Kontaktieren der immobilisierten mRNA und des synthetisierten cDNA-Moleküls mit RNAseH umfasst.

**27.** Verfahren nach Anspruch 24, 25 oder 26, worin zumindest eines der Desoxynucleotide eine detektierbare Markierung umfasst, worin das detektierbar markierte Desoxynucleotid in das synthetisierte Nucleinsäuremolekül inkorporiert ist.

**28.** Vorrichtung zur Immobilisierung und Modifizierung von Biomolekülen, die Folgendes umfasst:

eine Trenneinheit (302), wobei die Einheit (302) Folgendes umfasst:

ein magnetisches Joch (310) mit zumindest einer darin ausgebildeten Aussparung (312);
ein Paar Magnete (314), die jeweils in der zumindest einen Aussparung zur Bildung eines Abstands (316) dazwischen angeordnet sind, wobei der Abstand geeignet ist, eine Trennkammer darin aufzunehmen; und
eine steuerbare Wärme- und/oder Kühlungsquelle (332), die thermisch an das Magnetpaar (314) gekoppelt ist.

**29.** Vorrichtung nach Anspruch 28, die weiters zumindest eine Trennkammer umfasst, die eine benetzbare, wärmeleitfähige Durchflussmatrix aufweist, die in der Trennkammer enthalten ist.

**30.** Vorrichtung nach Anspruch 29, worin die benetzbare, wärmeleitfähige Durchflussmatrix (210) innen magnetisierbar ist.

**31.** Vorrichtung nach einem der Ansprüche 28 bis 30, die weiters eine Isolationsschicht (317) umfasst, die die Magneten und die steuerbare Wärmequelle trennt.

**32.** Vorrichtung nach einem der Ansprüche 28 bis 31, worin die steuerbare Wärmequelle jeweils auch als steuerbare Kühlungsquelle fungiert.

**33.** Vorrichtung nach einem der Ansprüche 28 bis 31, die weiters ein wärmeleitfähiges Element (338) umfasst, das die steuerbare Wärmequellen jeweils mit dem entsprechenden Magnetpaar thermisch verbindet.

**34.** Vorrichtung nach Anspruch 33, worin das wärmeleitfähige Element (338) konfiguriert ist, um eine Trennkammer zu kontaktieren, um Wärme in diese zu leiten.

**35.** Vorrichtung nach einem der Ansprüche 28 bis 34, worin zumindest eine der steuerbaren Wärmequellen einen Heizfilm (332) umfasst.

**36.** Vorrichtung nach einem der Ansprüche 28 bis 35, worin zumindest eine der steuerbaren Wärmequellen eine Wärmequelle vom Peltier-Typ umfasst.

**37.** Vorrichtung nach einem der Ansprüche 28 bis 36, worin zumindest eine der steuerbaren Wärmequellen eine Wärmequelle vom Widerstandstyp (432) umfasst.

**38.** Vorrichtung nach einem der Ansprüche 28 bis 37, worin die steuerbare Wärmequelle ein pneumatisches Heizsystem umfasst.

**39.** Vorrichtung nach einem der Ansprüche 28 bis 38, worin die steuerbare Wärmequelle ein hydraulisches Heizsystem umfasst.

**40.** Vorrichtung nach einem der Ansprüche 28 bis 39, worin zumindest eine der steuerbaren Wärmequellen ein flüssigkeitsbetriebenes Element umfasst, das geeignet ist, um Wärme von einer durch dieses zirkulierenden Flüssigkeit zu übertragen.

**41.** Vorrichtung nach einem der Ansprüche 28 bis 40, worin zumindest eine der steuerbaren Wärmequellen ein Strahlungsheizelement umfasst.

**42.** Vorrichtung nach Anspruch 41, worin das Strahlungsheizelement eine Infrarotlicht emittierende Diode umfasst.

**43.** Vorrichtung nach einem der Ansprüche 28 bis 42, worin zumindest eine der steuerbaren Wärmequellen ein Induktionsheizelement umfasst.

**44.** Vorrichtung nach Anspruch 43, worin jedes der Induktionsheizelemente eine Spule (1032) aus aufgewickeltem Draht umfasst.

**45.** Vorrichtung nach Anspruch 28 bis 44, die weiters ein Steuerelement (734) umfasst, das die steuerbare Wärme- und/oder Kühlungsquelle (332) mit einer Stromquelle (1030) verbindet, worin das Steuerelement dazu dient, die an jede der steuerbaren Wärmequellen gelieferte Menge Strom zu kontrollieren, um deren Temperatur zu steuern.

**46.** Vorrichtung nach einem der Ansprüche 28 bis 45, die weiters einen Rückmeldungssensor (348) umfasst, der mit jeder der steuerbaren Wärmequellen verbunden ist, um dem Steuerelement eine Rückmeldung in Bezug auf die Temperatur der jeweiligen steuerbaren Wärme- und/oder Kühlungsquelle bereitzustellen.

**47.** Vorrichtung nach Anspruch 46, worin die Rückmeldungssensoren jeweils ein Thermoelement umfasst.

**Revendications**

**1.** Procédé pour modifier une biomolécule, comprenant:

a) immobiliser une biomolécule liée à une particule magnétique sur un appareil de séparation magnétique comprenant au moins une chambre de séparation en appliquant un champ magnétique à une matrice magnétisable (210) dans la chambre de séparation, où l'appareil de séparation comprend en outre une unité de séparation (302) munie d'une source de chaleur et/ou de refroidissement contrôlable (332) pour contrôler la température dans la au moins une chambre de séparation *in situ,* et

b) modifier la biomolécule immobilisée, où une ou plusieurs étapes de modification sont exécutées à une température qui convient à la modification.

**2.** Procédé selon la revendication 1, comprenant en outre l'élution de la biomolécule modifiée de la chambre de séparation.

**3.** Procédé selon la revendication 1 ou 2, dans lequel la modification est une modification enzymatique avec au moins une première enzyme, et l'appareil est maintenu pendant une première période de temps à une première température à laquelle la première enzyme présente au moins 10% de son activité maximale.

**4.** Procédé selon la revendication 3, comprenant en outre:

c) modifier la biomolécule immobilisée avec une deuxième enzyme, où l'appareil est maintenu pendant une deuxième période de temps à une deuxième température à laquelle la deuxième enzyme présente au moins 10% de son activité maximale.

**5.** Procédé selon l'une quelconque des revendications précédentes, dans lequel la biomolécule comprend un polypeptide, et la modification est sélectionnée dans le groupe consistant en phosphorylation, déphosphorylation, nitrosylation, acétylation, déglycosylation, glycosylation, acylation, méthylation, ADP riboxlation, ubiquination, lipidation, carboxylation, hydroxylation; et nucléotidylation.

**6.** Procédé selon l'une quelconque des revendications précédentes, dans lequel la biomolécule comprend un polypeptide, et la modification est marquée avec un marqueur détectable.

**7.** Procédé selon la revendication 1 ou 2, dans lequel la biomolécule immobilisée comprend un polynucléotide, et la modification comprend l'hybridation à une deuxième biomolécule comprenant un polynucléotide comprenant une séquence de nucléotides qui est sensiblement complémentaire à au moins une portion du polynucléotide immobilisé.

**8.** Procédé selon la revendication 7, dans lequel la biomolécule immobilisée est un polynucléotide, et la modification comprend la synthétisation d'un polynucléotide comprenant une séquence de nucléotides qui est complémentaire à une séquence de nucléotides dans le polynucléotide immobilisé.

**9.** Procédé selon la revendication 1 ou 2, dans lequel la biomolécule immobilisée comprend un polynucléotide, et la modification est une modification enzymatique sélectionnée dans le groupe consistant en la synthèse d'un polynucléotide complémentaire au polynucléotide immobilisé, l'addition d'un nucléotide à l'extrémité 5' du polynucléotide immobilisé, l'addition d'un nucléotide à l'extrémité 3' du polynucléotide immobilisé, la ligation du polynucléotide à brin simple au polynucléotide immobilisé, la ligation d'un polynucléotide à brin double au polynucléotide immobilisé, le clivage du polynucléotide immobilisé à un site de reconnaissance d'endonucléase de restriction, le retrait d'un nucléotide du polynucléotide immobilisé, la synthèse d'un polypeptide utilisant le polynucléotide immobilisé comme matrice, et la méthylation d'une base d'un nucléotide du polynucléotide immobilisé.

**10.** Procédé selon la revendication 1 ou 2, dans lequel la biomolécule immobilisée comprend un polynucléotide, et la modification est une modification non enzymatique.

**11.** Procédé selon la revendication 1 ou 2, dans lequel la biomolécule immobilisée comprend un polynucléotide, et la modification comprend la liaison d'un polypeptide au polynucléotide immobilisé.

**12.** Procédé selon la revendication 1 ou 2, dans lequel la biomolécule immobilisée comprend un premier polypeptide, et la modification comprend la liaison d'un deuxième polypeptide au polypeptide immobilisé.

**13.** Procédé selon la revendication 1 ou 2, dans lequel la biomolécule immobilisée comprend un polynucléotide à brin double, et la modification comprend la mise en contact du polynucléotide immobilisé avec un polynucléotide à brin double d'environ 6 à environ 20 nucléotides en longueur, en présence d'une ligase d'ADN, à une température d'environ 16°C.

**14.** Procédé selon la revendication 1, où la biomolécule immobilisée comprend un polynucléotide, et où la particule magnétique contient, lié à celle-ci, un oligonucléotide qui est complémentaire à une portion de la biomolécule immobilisée et qui sert d'amorce à la synthèse d'un acide nucléique;
où à l'étape (b), la modification comprend

    i) mettre en contact le polynucléotide immobilisé avec une enzyme qui peut synthétiser une molécule d'acide nucléique, en présence de désoxynucléotides, où l'appareil est maintenu pendant une période de temps à une température à laquelle l'enzyme présente au moins 10% de son activité maximale; et
    ii) synthétiser une molécule d'acide nucléique, en utilisant le polynucléotide immobilisé comme matrice.

**15.** Procédé selon la revendication 14, où au moins un désoxynucléotide comprend un marqueur détectable, et où la molécule d'acide nucléique synthétisée comprend le au moins un désoxynucléotide marqué d'une manière détectable.

**16.** Procédé selon la revendication 14 ou 15, dans lequel le polynucléotide immobilisé est une molécule d'ARNm, où l'enzyme est une transcryptase inverse, où l'étape (ii) est exécutée à une température d'environ 32°C à environ 42°C et où la molécule d'acide nucléique synthétisée est une molécule d'ADNc.

**17.** Procédé selon la revendication 16, comprenant en outre:

    c) mettre en contact la molécule d'ADNc avec RNaseR à une température d'environ 37°C; et
    d) éluer la molécule d'ADNc.

**18.** Procédé selon la revendication 1, dans lequel la biomolécule immobilisée comprend un polynucléotide;
où à l'étape (b), la modification comprend

    i) mettre en contact le polynucléotide immobilisé avec une première amorce d'oligonucléotide et une enzyme qui peut synthétiser une molécule d'acide nucléique, en présence de désoxynucléotides, sous des conditions qui conviennent à la synthèse d'un acide nucléique; et
    ii) synthétiser une molécule d'acide nucléique, en utilisant le polynucléotide immobilisé comme matrice.

**19.** Procédé selon la revendication 18, où durant l'étape (b), l'appareil est maintenu pendant une période de temps à une température à laquelle l'enzyme présente au moins 10% de son activité maximale.

**20.** Procédé selon la revendication 18 ou 19, dans lequel l'étape (i) est exécutée à une température d'environ 55°C et/ou où l'étape (ii) est exécutée à une température d'environ 60°C à environ 72°C.

**21.** Procédé selon la revendication 18, 19 ou 20, comprenant en outre:

    (c) chauffer la chambre de séparation à une température pour dénaturer l'acide nucléique synthétisé;
    (d) mettre en contact la molécule d'acide nucléique synthétisée avec une deuxième amorce d'oligonucléotide qui s'hybride à une région dans la molécule d'acide nucléique synthétisée;
    (e) refroidir la chambre de séparation à une température suffisante pour permettre l'hybridation de la deuxième amorce à la molécule d'acide nucléique synthétisée de sorte que l'hybridation se produit; et
    (f) amener la chambre de séparation à une température qui convient pour que la synthèse d'un deuxième acide nucléique à partir de ladite deuxième amorce se produise.

**22.** Procédé selon la revendication 21, dans lequel l'échauffement à l'étape (c) est d'environ 90°C à environ 96°C; et/ou le refroidissement (e) est jusqu'à environ 55°C; et/ou la synthèse (f) est d'environ 60°C à environ 72°C.

**23.** Procédé selon la revendication 21 ou 22 comprenant en outre la répétition des cycles des étapes de dénaturation, refroidissement et synthèse de 2 à environ 30 fois.

**24.** Procédé selon la revendication 1, dans lequel la biomolécule immobilisée comprend un polynucléotide comprenant un poly(A)tractus, et la particule magnétique est liée à une oligo-dT molécule d'environ 6 nucléotides à environ 30 nucléotides;
où dans l'étape (b), la modification comprend

    i) mettre en contact le polynucléotide immobilisé avec une enzyme qui peut synthétiser une molécule d'acide nucléique, en présence de désoxynucléotides, où l'appareil est maintenu pendant une période de temps à une température à laquelle l'enzyme présente au moins 10% de son activité maximale; et

ii) synthétiser une molécule d'acide nucléique en utilisant le polynucléotide immobilisé comme matrice.

25. Procédé selon la revendication 24, où le polynucléotide immobilisé est une molécule d'ARNm, et la molécule d'acide nucléique synthétisée est une molécule d'ADNc.

26. Procédé selon la revendication 25, comprenant en outre la mise en contact du ARNm immobilisé et de la molécule d'ADNc synthétisée avec ARNseH.

27. Procédé selon la revendication 24, 25 ou 26, où au moins un des désoxynucléotides comprend un marqueur détectable, où le désoxynucléotide marqué d'une manière détectable est incorporé dans la molécule d'acide nucléique synthétisée.

28. Appareil pour immobiliser et modifier des biomolécules, comprenant:

une unité de séparation (302), l'unité (302) comprenant:

un étrier magnétique (310) ayant au moins une encoche (312) formée dans celui-ci;
une paire d'aimants (314) placés dans chacune desdites au moins une encoche pour former un espace (316) entre celles-ci, ledit espace étant apte à recevoir une chambre de séparation à l'intérieur; et
une source de chaleur et/ou source de refroidissement contrôlable (332) couplée thermiquement à chaque paire d'aimants précités (314).

29. Appareil selon la revendication 28, comprenant en outre au moins une chambre de séparation ayant une matrice de conduction de chaleur apte à être mouillée, à écoulement traversant, se trouvant dans chaque chambre de séparation précitée.

30. Appareil selon la revendication 29, où ladite matrice de conduction de chaleur apte à être mouillée, à écoulement traversant (210), peut être aimantée intérieurement.

31. Appareil selon l'une quelconque des revendications 28 à 30, comprenant en outre une couche d'isolation (317) séparant lesdits éléments et ladite source de chaleur contrôlable.

32. Appareil selon l'une quelconque des revendications 28 à 31, dans lequel chaque source de chaleur contrôlable précitée fonctionne également comme source de refroidissement contrôlable.

33. Appareil selon l'une quelconque des revendications 28 à 31, comprenant en outre un élément de conduction de chaleur (338) reliant thermiquement chaque source de chauffage contrôlable précitée à ladite paire respective d'aimants.

34. Appareil selon la revendication 33, où chaque élément de conduction de chaleur précitée (338) est configuré pour venir en contact avec une chambre de séparation pour conduire la chaleur à celle-ci.

35. Appareil selon l'une quelconque des revendications 28 à 34, où au moins une desdites sources de chaleur contrôlables comprend un film de chauffage (332).

36. Appareil selon l'une quelconque des revendications 28 à 35, dans lequel au moins une desdites sources de chaleur contrôlables comprend une source de chaleur de type Peltier.

37. Appareil selon l'une quelconque des revendications 28 à 36, où au moins une desdites sources de chaleur contrôlables comprend une source de chaleur du type de résistance de puissance (432).

38. Appareil selon l'une quelconque des revendications 28 à 37, où ladite source de chaleur contrôlable comprend un système de chauffage pneumatique.

39. Appareil selon l'une quelconque des revendications 28 à 38, où ladite source de chaleur contrôlable comprend un système de chauffage hydraulique.

**40.** Appareil selon l'une quelconque des revendications 28 à 39, dans lequel au moins une desdites sources de chaleur contrôlables comprend un élément mené par du liquide apte à transférer la chaleur d'un liquide circulant à travers celui-ci.

**41.** Appareil selon l'une quelconque des revendications 28 à 40, dans lequel au moins une desdites sources de chaleur contrôlables comprend un élément de chauffage rayonnant.

**42.** Appareil selon la revendication 41, où chaque élément de chauffage rayonnant comprend une diode émettrice de lumière infrarouge.

**43.** Appareil selon l'une quelconque des revendications 28 à 42, où au moins une desdites sources de chaleur contrôlables comprend un élément de chauffage inductif.

**44.** Appareil selon la revendication 43, dans lequel chaque élément de chauffage inductif précité comprend une bobine (1032) de fil enroulé.

**45.** Appareil selon l'une quelconque des revendications 28 à 44, comprenant en outre un dispositif de commande (734) couplant chacune parmi ladite source de chaleur et/ou de refroidissement contrôlable (332) à une source de puissance (1030), où ledit contrôleur fonctionne pour commander une quantité de puissance délivrée à chaque source de chaleur contrôlable pour contrôler une température de celle-ci.

**46.** Appareil selon l'une quelconque des revendications 28 à 45, comprenant en outre un capteur de réaction (348) associé à chacune desdites sources de chaleur contrôlables pour fournir une réaction audit contrôleur se rapportant à une température de ladite source de chaleur et/ou de refroidissement contrôlable respective.

**47.** Appareil selon la revendication 46, dans lequel chaque capteur de réaction comprend un thermocouple.

FIG. 1

FIG. 2

FIG. 3A

FIG. 3B

POWER SUPPLY

EP 1 450 960 B1

FIG. 4A

FIG. 4B

POWER SUPPLY

FIG. 5A

400

342

344

334

320

438

432

402

POWER SUPPLY

330

346    346    200    316    314    310

440

438

FIG. 5B

440

438

FIG. 5C

EP 1 450 960 B1

FIG. 6B

FIG. 6A

EP 1 450 960 B1

FIG. 7A

FIG. 7B

FIG. 8A

FIG. 8B

FIG. 9A

FIG. 9B

# FIG. 10

FIG. 11

EP 1 450 960 B1

FIG. 12

**EP 1 450 960 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5411863 A
- US 4452773 A
- US 4230685 A
- US 6020210 A
- US 6159378 A
- WO 0117687 A
- US 6159689 A
- US 4738773 A
- US 4664796 A
- US 4375407 A
- US 5711871 A
- US 5705059 A
- US 5691208 A
- US 5543289 A
- US 5779892 A
- US 6020201 A
- US 4770183 A
- US 09556179 B

- WO 9816427 A
- WO 9855495 A
- WO 0021556 A
- US 4849513 A
- US 5015733 A
- US 5118800 A
- US 5118802 A
- US 5594115 A
- US 5284933 A
- US 5047513 A
- US 5310663 A
- US 5707829 A, Sambrook
- US 5695937 A
- US 5866330 A
- US 6221600 B
- US 6261782 B
- US 6297017 B

**Non-patent literature cited in the description**

- **Lansdorp ; Thomas.** *Mol. Immunol.,* 1990, vol. 27, 659
- Current Protocols in Nucleic Acid Chemistry. John Wiley & Sons, 1999
- **Kisakurek et al.** Frontiers in Nucleic Acid Chemistry. John Wiley & Sons, 2000
- **Haralambidis et al.** *Nucl. Acid Res.,* 1990, vol. 18, 493-499
- **Haralambidis et al.** *Nucl. Acid Res.,* 1990, vol. 18, 501-505
- **Zuckermann et al.** *Nucl. Acids Res.,* 1987, vol. 15, 5305-5321
- **Nelson et al.** *Nucl. Acids. Res.,* 1989, vol. 17, 1781-1794
- **Benoit et al.** *Neuromethods,* 1987, vol. 6, 43-72
- **Sinah et al.** Oligonucleotide Analogues: A Practical Approach. IRL Press, 1991
- **Tung et al.** *Bioconj. Chem.I,* 1991, vol. 2, 464-465
- **Agrawal et al.** *Nucleic Acids Res.,* 1986, vol. 14, 6227-6245
- **Bischoff et al.** *Anal. Biochem.,* 1987, vol. 164, 336-344
- **Yanagawa et al.** *Nucleic Acids Symp. Ser.,* 1988, vol. 19, 189-192

- **Grabarek et al.** *Anal. Biochem.,* 1990, vol. 185, 131-135
- **Boujrad et al.** *Proc. Natl. Acad. Sci. USA,* 1993, vol. 90, 5728-5731
- **O'Shannessy et al.** *J. Applied Biochem.,* 1985, vol. 7, 347-355
- **Jones.** The Chemical Synthesis of Peptides. Clarendon Press, 1994
- Short Protocols in Molecular Biology. Wiley & Sons, 1999
- **Sambrook ; Maniatis ; Fritsch.** Molecular cloning: A laboratory manual. Cold Spring Harbor Laboratory, 1989
- Current Protocols in Molecular Biology. John Wiley & Sons, Inc, 1995
- **Edge et al.** *Nature,* 1981, vol. 292, 756
- **Duckworth et al.** *Nucleic Acids Res,* 1981, vol. 9, 1691
- **Beaucage et al.** *Tet. Letts,* 1981, vol. 22, 1859
- **Greg T. Hermanson.** Bioconjugate Techniques. Academic Press
- **Sambrook ; Russell.** Molecular Cloning. Cold Spring Harbor Laboratory Press
- A Practical Approach. Oxford Univ. Press, 1995
- Handbook of Proteolytic Enzymes. Academic Press, 1998

53

- **Sommergruber et al.** *Virol.,* 1994, vol. 198, 741-745
- **Schultheiss et al.** *J. Virol.,* 1995, vol. 69, 1727-1733
- **Wang et al.** *Biochem: Biophys. Res. Comm.,* 1997, vol. 235, 562-566
- **Walker et al.** *Biotechnol.,* 1994, vol. 12, 601-605
- **Velculescu et al.** *Science,* 1995, vol. 270, 484-7